# EUROPEAN PATENT APPLICATION

(11) **EP 4 692 790 A1**
(43) Date of publication of application: **11.02.2026**
(21) Application number: 24779435.7
(22) Date of filing: 12.03.2024
(51) Int. Cl.: G01N 33/48, G01N 33/15, G01N 33/50, G06Q 50/04, G16H 10/40, G06T 7/00, G16H 20/10, G16H 30/20, G16H 30/40, G16H 40/63, G16H 40/67, G16H 50/20, G16H 50/70

(54) **DRUG DISCOVERY ASSISTANCE DEVICE, METHOD FOR OPERATING DRUG DISCOVERY ASSISTANCE DEVICE, AND PROGRAM FOR OPERATING DRUG DISCOVERY ASSISTANCE DEVICE**

(30) Priority: 31.03.2023 JP 2023057992
(71) Applicant: FUJIFILM Corporation, Tokyo 106-8620 (JP)
(72) Inventor: TOMINAGA, Shunsuke, Ashigarakami-gun, Kanagawa 258-8577 (JP)
(74) Representative: HGF
(86) International application number: PCT/JP2024/009658
(87) International publication number: WO 2024/203306

(57) **Abstract**

A drug discovery support device includes a processor. The processor is configured to obtain a specimen image of a tissue specimen of an organ of a subject subjected to an evaluation test of a candidate substance; detect, from among portions of the specimen image, one or more estimated morphological abnormality portions where a morphological abnormality is estimated to have occurred; derive determination reference information from a detection result of each of the one or more estimated morphological abnormality portions; determine, based on the determination reference information, whether the one or more estimated morphological abnormality portions have been overdetected and whether the one or more estimated morphological abnormality portions have been underdetected; in response to a determination being made that the one or more estimated morphological abnormality portions have been overdetected, present to a user first cause identification reference information contributing to identifying a cause of the one or more estimated morphological abnormality portions having been overdetected; and in response to a determination being made that the one or more estimated morphological abnormality portions have been underdetected, present to the user second cause identification reference information contributing to identifying a cause of the one or more estimated morphological abnormality portions having been underdetected.

## Description

### BACKGROUND OF THE INVENTION

### 1. Field of the Invention

The technology of the present disclosure relates to a drug discovery support device, a method for operating a drug discovery support device, and a program for operating a drug discovery support device.

### 2. Description of the Related Art

In the field of drug discovery, a test is conducted in which a candidate substance for a drug is administered to a subject such as a rat, and the efficacy and toxicity of the candidate substance are evaluated. In such an evaluation test, a specimen image of a tissue specimen of an organ (a brain specimen, a liver specimen, a heart specimen, or the like) collected by autopsying a subject is used.

For example, JP2022-007281A describes the technology described below. That is, a specimen image to be analyzed is subjected to segmentation to extract partial regions (regions corresponding to specific features occurring in a specific disease, or the like). Subsequently, feature quantities obtained by quantifying characteristics such as the morphology of a tissue specimen in the partial regions (feature quantities, color feature quantities, shape feature quantities, or the like output from a neural network) are calculated. Subsequently, the specimen image on which information on the partial regions is superimposed is displayed to a user, and the user is caused to designate partial regions belonging to the same category. Subsequently, auxiliary information on the feature quantities related to the category (such as the degree of contribution of each feature quantity when the partial regions are classified on a category basis) is generated and displayed to the user.

### SUMMARY OF THE INVENTION

In the evaluation of a candidate substance for a drug, a morphological abnormality occurred in a tissue specimen in a specimen image is detected. Hitherto, an expert such as a pathologist observes a specimen image to detect a portion where a morphological abnormality is estimated to have occurred (hereinafter referred to as an estimated morphological abnormality portion). With the recent progress in an image analysis technology, a technology for automatically detecting an estimated morphological abnormality portion without troubling an expert has been developed.

When an estimated morphological abnormality portion is to be detected by image analysis, attention is to be paid that the following cases may occur: a case in which a portion where no morphological abnormality has occurred is erroneously determined to be an estimated morphological abnormality portion, and as a result the estimated morphological abnormality portion is overdetected; and a converse case in which a portion where a morphological abnormality has occurred is erroneously determined not to be an estimated morphological abnormality portion, and as a result the estimated morphological abnormality portion is underdetected.

There are various possible causes for overdetecting or underdetecting an estimated morphological abnormality portion. If the causes can be identified and an appropriate measure can be taken, the detection accuracy of a morphological abnormality occurred in a tissue specimen in a specimen image can be increased. However, such a technology has not been proposed in the related art including JP2022-007281A.

One embodiment according to the technology of the present disclosure provides a drug discovery support device, a method for operating a drug discovery support device, and a program for operating a drug discovery support device that are capable of contributing to increasing the detection accuracy of a morphological abnormality occurred in a tissue specimen in a specimen image.

A drug discovery support device according to the present disclosure includes a processor. The processor is configured to obtain a specimen image of a tissue specimen of an organ of a subject subjected to an evaluation test of a candidate substance; detect, from among portions of the specimen image, one or more estimated morphological abnormality portions where a morphological abnormality is estimated to have occurred; derive determination reference information from a detection result of each of the one or more estimated morphological abnormality portions; determine, based on the determination reference information, whether the one or more estimated morphological abnormality portions have been overdetected and whether the one or more estimated morphological abnormality portions have been underdetected; in response to a determination being made that the one or more estimated morphological abnormality portions have been overdetected, present to a user first cause identification reference information contributing to identifying a cause of the one or more estimated morphological abnormality portions having been overdetected; and in response to a determination being made that the one or more estimated morphological abnormality portions have been underdetected, present to the user second cause identification reference information contributing to identifying a cause of the one or more estimated morphological abnormality portions having been underdetected.

Preferably, the processor is configured to obtain the specimen image, the specimen image including a single specimen image; detect the one or more estimated morphological abnormality portions from the single specimen image; and derive, as the determination reference information, a numerical value representing a spatial disposition state of the one or more estimated morphological abnormality portions in the single specimen image.

Preferably, the processor is configured to compare the numerical value with a determination threshold value set in advance, and thus determine whether the one or more estimated morphological abnormality portions have been overdetected and whether the one or more estimated morphological abnormality portions have been underdetected.

Preferably, the numerical value is a numerical value related to the number of the one or more estimated morphological abnormality portions.

Preferably, the processor is configured to obtain the specimen image, the specimen image including multiple specimen images; detect the one or more estimated morphological abnormality portions from each of the multiple specimen images; and derive, as the determination reference information, a distribution of numerical values each representing a spatial disposition state of the one or more estimated morphological abnormality portions in a corresponding one of the multiple specimen images.

Preferably, the multiple specimen images are images of a tissue specimen of multiple subjects belonging to the same group, and the processor is configured to detect an outlier from among the numerical values constituting the distribution, and thus determine whether the one or more estimated morphological abnormality portions have been overdetected and whether the one or more estimated morphological abnormality portions have been underdetected.

Preferably, the multiple specimen images are images of a tissue specimen of multiple subjects belonging to different groups, and the processor is configured to derive the distribution for each of the different groups; and detect an outlier from among the numerical values constituting one distribution of the multiple distributions derived individually for the different groups, refer to a distribution other than the one distribution, and thus determine whether the one or more estimated morphological abnormality portions have been overdetected and whether the one or more estimated morphological abnormality portions have been underdetected.

Preferably, the different groups are an administration group to which the candidate substance is administered and a control group to which the candidate substance is not administered, or an administration group to which the candidate substance is administered and an administration group to which a candidate substance identical or similar to the candidate substance is administered in a past evaluation test.

Preferably, the numerical values are each a numerical value related to the number of the one or more estimated morphological abnormality portions.

Preferably, the processor is configured to obtain the specimen image, the specimen image including multiple specimen images of a tissue specimen of multiple subjects belonging to different groups; detect the one or more estimated morphological abnormality portions from each of the multiple specimen images; and derive, as the determination reference information, for each of the different groups, a representative value of numerical values each representing a spatial disposition state of the one or more estimated morphological abnormality portions in a corresponding one of the multiple specimen images.

Preferably, the processor is configured to compare the representative value of each of the different groups with an ideal value of the numerical values that is set in advance for a corresponding one of the different groups, and thus determine whether the one or more estimated morphological abnormality portions have been overdetected and whether the one or more estimated morphological abnormality portions have been underdetected.

Preferably, the different groups are an administration group to which the candidate substance is administered and a control group to which the candidate substance is not administered.

Preferably, the administration group includes multiple sub-administration groups that are different from each other in a dose of the candidate substance.

Preferably, the numerical values are each a numerical value related to the number of the one or more estimated morphological abnormality portions.

Preferably, multiple types of artifact have a possibility of being erroneously detected as the one or more estimated morphological abnormality portions, and the processor is configured to identify a type of the artifact that has a possibility of having been erroneously detected as the one or more estimated morphological abnormality portions; and present, as the first cause identification reference information and the second cause identification reference information, information regarding the type to the user.

Preferably, the processor is configured to present, as the first cause identification reference information and the second cause identification reference information, information regarding a color of the specimen image and information regarding a color of a reference specimen image of a tissue specimen regarded to be normal to the user.

Preferably, the specimen image includes a single specimen image, the one or more estimated morphological abnormality portions include multiple estimated morphological abnormality portions, and the processor is configured to, in response to a determination being made that the one or more estimated morphological abnormality portions have been overdetected, perform a clustering process of defining a cluster to which each of the multiple estimated morphological abnormality portions detected from the single specimen image belongs; and present, as the first cause identification reference information, multiple cluster images that are generated by processing the specimen image, that reflect a result of the clustering process, and that enable multiple clusters to be identified by a display format set in advance for each of the multiple clusters to the user.

Preferably, the processor is configured to propose to the user and/or perform a suppression process of suppressing overdetection or underdetection of the one or more estimated morphological abnormality portions.

Preferably, the suppression process includes at least one of a process of changing a detection threshold value that is to be used to detect the one or more estimated morphological abnormality portions; a process of correcting a color of the specimen image; or a process of changing a resolution of the specimen image in detection of the one or more estimated morphological abnormality portions.

Preferably, the processor is configured to propose to the user and/or perform an exclusion process of excluding, from a target of the evaluation test, a portion of an artifact that has a possibility of having been erroneously detected as the one or more estimated morphological abnormality portions.

Preferably, the processor is configured to handle each of multiple patch images obtained by dividing the specimen image as one of the portions; and compare feature quantities obtained by inputting the patch images to a machine learning model with reference feature quantities obtained by inputting reference patch images of a tissue specimen regarded to be normal to the machine learning model, and thus detect the one or more estimated morphological abnormality portions.

A method for operating a drug discovery support device according to the present disclosure includes obtaining a specimen image of a tissue specimen of an organ of a subject subjected to an evaluation test of a candidate substance; detecting, from among portions of the specimen image, one or more estimated morphological abnormality portions where a morphological abnormality is estimated to have occurred; deriving determination reference information from a detection result of each of the one or more estimated morphological abnormality portions; determining, based on the determination reference information, whether the one or more estimated morphological abnormality portions have been overdetected and whether the one or more estimated morphological abnormality portions have been underdetected; in response to a determination being made that the one or more estimated morphological abnormality portions have been overdetected, presenting to a user first cause identification reference information contributing to identifying a cause of the one or more estimated morphological abnormality portions having been overdetected; and in response to a determination being made that the one or more estimated morphological abnormality portions have been underdetected, presenting to the user second cause identification reference information contributing to identifying a cause of the one or more estimated morphological abnormality portions having been underdetected.

A program for operating a drug discovery support device according to the present disclosure causes a computer to execute a process including obtaining a specimen image of a tissue specimen of an organ of a subject subjected to an evaluation test of a candidate substance; detecting, from among portions of the specimen image, one or more estimated morphological abnormality portions where a morphological abnormality is estimated to have occurred; deriving determination reference information from a detection result of each of the one or more estimated morphological abnormality portions; determining, based on the determination reference information, whether the one or more estimated morphological abnormality portions have been overdetected and whether the one or more estimated morphological abnormality portions have been underdetected; in response to a determination being made that the one or more estimated morphological abnormality portions have been overdetected, presenting to a user first cause identification reference information contributing to identifying a cause of the one or more estimated morphological abnormality portions having been overdetected; and in response to a determination being made that the one or more estimated morphological abnormality portions have been underdetected, presenting to the user second cause identification reference information contributing to identifying a cause of the one or more estimated morphological abnormality portions having been underdetected.

According to the technology of the present disclosure, it is possible to provide a drug discovery support device, a method for operating a drug discovery support device, and a program for operating a drug discovery support device that are capable of contributing to increasing the detection accuracy of a morphological abnormality occurred in a tissue specimen in a specimen image.

### BRIEF DESCRIPTION OF THE DRAWINGS

Fig. 1 is a diagram illustrating a process of an evaluation test, specimen images, and a drug discovery support device;
Fig. 2 is a diagram illustrating an administration group and a control group;
Fig. 3 is a block diagram illustrating a computer constituting the drug discovery support device;
Fig. 4 is a block diagram illustrating processing units of a central processing unit (CPU) of the drug discovery support device;
Fig. 5 is a diagram illustrating an original image and an analysis image of a specimen image;
Fig. 6 is a diagram illustrating patch images obtained by dividing a specimen image;
Fig. 7 is a diagram illustrating a state in which feature quantities are extracted from patch images by a feature quantity extractor;
Fig. 8 is a diagram illustrating the structure of the feature quantity extractor;
Fig. 9 is a diagram illustrating a process in a learning phase of an autoencoder;
Fig. 10 is a diagram illustrating a past control group and the formation of learning reference patch images;
Fig. 11 is a diagram illustrating a state in which reference feature quantities are extracted from reference patch images by the feature quantity extractor;
Fig. 12 is a diagram illustrating a graph in which reference feature quantities are plotted in a feature quantity space, and detection reference information;
Fig. 13 is a diagram illustrating a distance between a position of a feature quantity and a representative position of reference feature quantities;
Fig. 14 is a diagram illustrating a process performed by a detection unit and a detection result;
Fig. 15 is a diagram illustrating a process performed by the detection unit and a detection result;
Fig. 16 is a diagram illustrating a process performed by a derivation unit and determination reference information;
Fig. 17 is a diagram illustrating a process performed by a determination unit and a generation unit and a determination result;
Fig. 18 is a diagram illustrating a process performed by the determination unit and the generation unit and a determination result;
Fig. 19 is a diagram illustrating generation reference information;
Fig. 20 is a flowchart illustrating a procedure of a process performed by the generation unit;
Fig. 21 is a diagram illustrating a process performed by the generation unit;
Fig. 22 is a diagram illustrating a process performed by the generation unit and cause identification reference information;
Fig. 23 is a diagram illustrating a process performed by the generation unit and cause identification reference information;
Fig. 24 is a diagram illustrating a process performed by the generation unit and cause identification reference information;
Fig. 25 is a diagram illustrating a process performed by the generation unit;
Fig. 26 is a diagram illustrating cause identification reference information;
Fig. 27 is a diagram illustrating a target designation screen;
Fig. 28 is a diagram illustrating an analysis result display screen;
Fig. 29 is a diagram illustrating an information display screen;
Fig. 30 is a diagram illustrating an information display screen;
Fig. 31 is a diagram illustrating an information display screen;
Fig. 32 is a diagram illustrating another example of a message promoting the execution of an improvement process;
Fig. 33 is a diagram illustrating another example of a message promoting the execution of an improvement process;
Fig. 34 is a flowchart illustrating a procedure of a process performed by the drug discovery support device;
Fig. 35 is a block diagram illustrating processing units of a CPU of a drug discovery support device according to a second embodiment;
Fig. 36 is a diagram illustrating a process performed by a CPU and determination reference information according to a third embodiment;
Fig. 37 is a diagram illustrating another example of the process performed by the CPU and determination reference information according to the third embodiment;
Fig. 38 is a diagram illustrating still another example of the process performed by the CPU and determination reference information according to the third embodiment;
Fig. 39 is a diagram illustrating still another example of the process performed by the CPU and determination reference information according to the third embodiment;
Fig. 40 is a diagram illustrating a process performed by a CPU and determination reference information according to a fourth embodiment;
Fig. 41 is a diagram illustrating a process performed by a determination unit according to the fourth embodiment;
Fig. 42 is a diagram illustrating another example of a process performed by the derivation unit and the determination unit and determination reference information;
Fig. 43 is a diagram illustrating another example of the process performed by the derivation unit and the determination unit and determination reference information;
Fig. 44 is a diagram illustrating still another example of the process performed by the derivation unit and the determination unit and determination reference information;
Fig. 45 is a diagram illustrating a fifth embodiment of handling a specimen image obtained by imaging a specimen slide on which tissue specimens of multiple types of organs are placed; and
Fig. 46 is a diagram illustrating a state in which feature quantities are extracted from patch images obtained by dividing a heart specimen by a feature quantity extractor for a heart specimen.

### DESCRIPTION OF THE PREFERRED EMBODIMENTS

### First Embodiment

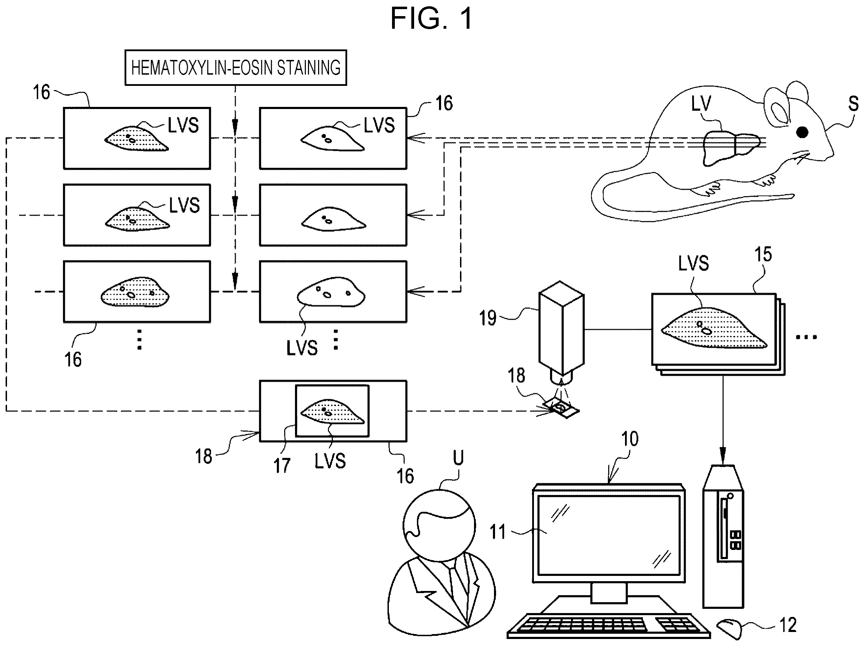

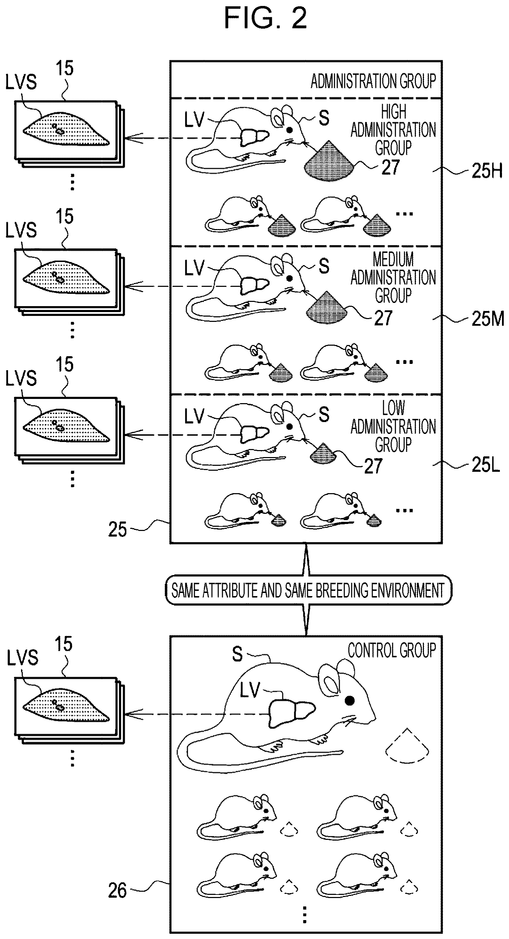

As illustrated in Fig. 1 as an example, a drug discovery support device 10 according to the present disclosure is used to evaluate the efficacy and toxicity of a candidate substance 27 (see Fig. 2) for a drug. The drug discovery support device 10 is, for example, a desktop personal computer, and includes a display 11 that displays various screens, and an input device 12 such as a keyboard, a mouse, a touch panel, and/or a microphone for voice input. The drug discovery support device 10 is installed in, for example, a drug development facility, and is operated by a user U such as a drug discovery staff member engaged in the development of a drug in the drug development facility. The drug discovery staff member includes a pathologist or the like.

The drug discovery support device 10 receives specimen images 15. The specimen images 15 are images for evaluating the efficacy and toxicity of the candidate substance 27. The specimen images 15 are generated by, for example, the following procedure. First, a subject S such as a rat prepared for evaluating the candidate substance 27 is autopsied, and multiple tissue specimens of transverse sections of an organ, a liver LV in this case, of the subject S (hereinafter referred to as liver specimens LVS) are collected. Subsequently, the collected liver specimens LVS are each applied to a glass slide 16, and then the liver specimens LVS are stained, here, with a hematoxylin-eosin stain. Subsequently, the stained liver specimens LVS are each covered with a coverslip 17 to complete specimen slides 18. Subsequently, the specimen slides 18 are each set to an image capturing device 19 such as a digital optical microscope, and the image capturing device 19 captures specimen images 15. The specimen images 15 obtained in this manner each have the whole liver specimen LVS. Thus, the specimen images 15 are called whole slide images (WSIs). The specimen images 15 are each given subject identification data (ID) for uniquely identifying the subject S, specimen image ID for uniquely identifying the specimen image 15, the date and time of capturing, and so forth. The tissue specimen is also called a tissue section. The staining may be staining with a hematoxylin stain alone, staining with a nuclear fast red stain, or the like.

As illustrated in Fig. 2 as an example, the subject S includes those belonging to an administration group 25 and those belonging to a control group 26. The administration group 25 is constituted by multiple subjects S to which the candidate substance 27 is administered. The administration group 25 is further grouped into a high administration group 25H, a medium administration group 25M, and a low administration group 25L in accordance with a dose of the candidate substance 27. As a result of grouping the administration group 25 into the high administration group 25H, the medium administration group 25M, and the low administration group 25L, it is possible to determine the influence of the dose of the candidate substance 27 on the subjects S. The high administration group 25H, the medium administration group 25M, and the low administration group 25L are an example of "sub-administration groups" according to the technology of the present disclosure. The sub-administration groups are not limited to the exemplified three groups of the high administration group 25H, the medium administration group 25M, and the low administration group 25L, and may be two groups of the high administration group 25H and the low administration group 25L, or four or more groups.

In contrast to the administration group 25, the control group 26 is constituted by multiple subjects S to which the candidate substance 27 is not administered. The number of subjects S constituting each of the high administration group 25H, the medium administration group 25M, and the low administration group 25L is the same as the number of subjects S constituting the control group 26, for example, about 5 to 10. The subjects S constituting each of the high administration group 25H, the medium administration group 25M, and the low administration group 25L and the subjects S constituting the control group 26 have the same attribute and are placed under the same breeding environment. The same attribute is, for example, the same age in weeks, the same sex, and/or the same genetic strain. The same genetic strain is, for example, the same ancestor before the fifth generation and/or the same gene sequence in a specific region. The same attribute also includes the same constituent ratio of ages in weeks, the same constituent ratio of sexes (five males and five females, for example), and/or the same constituent ratio of genetic strains. The same breeding environment is, for example, the same feed to be given, the same temperature and humidity of the breeding space, and/or the same area of the breeding space. The term "same" in the same breeding environment refers to not only completely the same, but also the same in a sense including an error that is generally accepted in the technical field to which the technology of the present disclosure pertains and that does not contradict the gist of the technology of the present disclosure.

Multiple specimen images 15 are obtained from a single subject S. Thus, the number of specimen images 15 obtained from each group is equal to the number obtained by multiplying the number of specimen images 15 obtained from a single subject S by the number of subjects S. For example, when the number of specimen images 15 obtained from a single subject S is 100 and the number of subjects S constituting each group is 10, 1000 (= 100×10) specimen images 15 are obtained from each group.

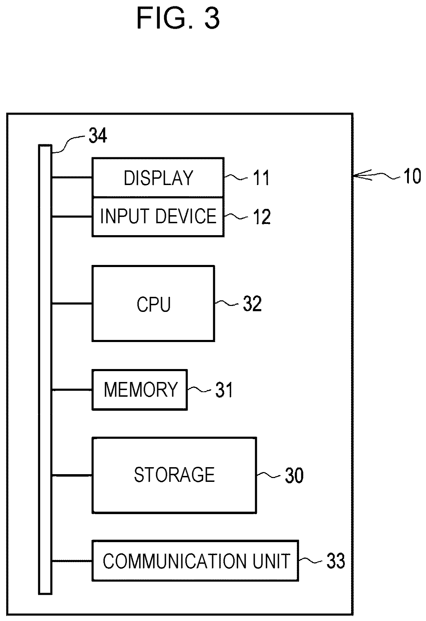

As illustrated in Fig. 3 as an example, the computer constituting the drug discovery support device 10 includes, in addition to the display 11 and the input device 12 described above, a storage 30, a memory 31, a central processing unit (CPU) 32, and a communication unit 33. These are connected to each other via a bus line 34.

The storage 30 is a hard disk drive built in the computer constituting the drug discovery support device 10 or connected to the computer via a cable or a network. Alternatively, the storage 30 is a disk array including multiple hard disk drives. The storage 30 stores a control program such as an operating system, various application programs, and various data associated with these programs. Alternatively, a solid-state drive may be used instead of the hard disk drive.

The memory 31 is a work memory for the CPU 32 to execute processing. The CPU 32 loads a program stored in the storage 30 into the memory 31 and executes processing in accordance with the program. Accordingly, the CPU 32 controls the individual units of the computer in a centralized manner. The CPU 32 is an example of "a processor" according to the technology of the present disclosure. The memory 31 may be built in the CPU 32. The communication unit 33 controls transmission of various pieces of information to an external device such as the image capturing device 19.

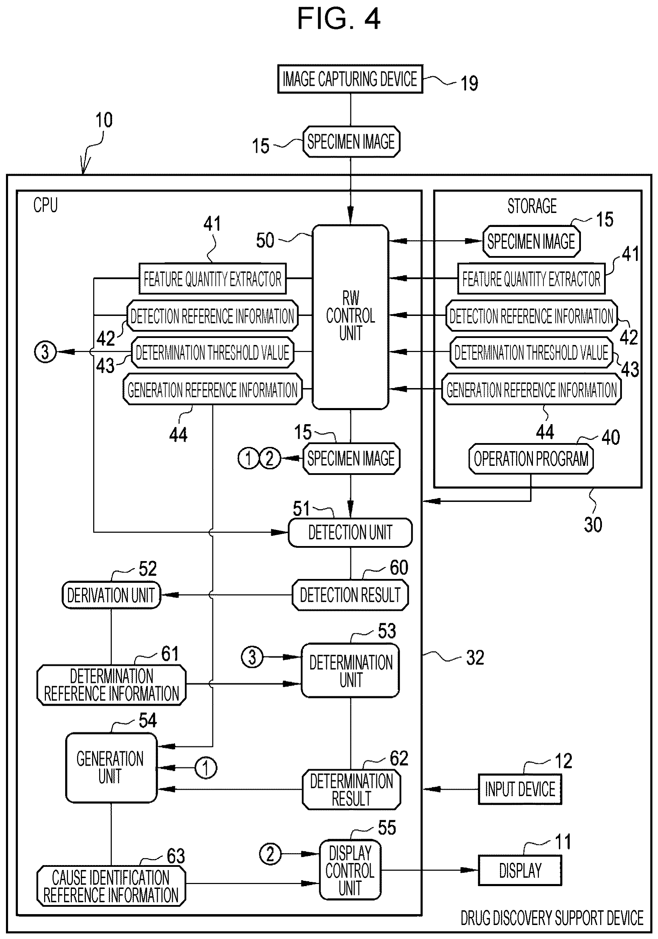

As illustrated in Fig. 4 as an example, the storage 30 of the drug discovery support device 10 stores an operation program 40. The operation program 40 is an application program for causing the computer to function as the drug discovery support device 10. That is, the operation program 40 is an example of "a program for operating a drug discovery support device" according to the technology of the present disclosure. The storage 30 also stores a feature quantity extractor 41, detection reference information 42, a determination threshold value 43, generation reference information 44, and so forth. The feature quantity extractor 41 is an example of "a machine learning model" according to the technology of the present disclosure.

Upon the operation program 40 being started, the CPU 32 of the computer constituting the drug discovery support device 10 cooperates with the memory 31 and so forth to function as a read/write (hereinafter abbreviated as RW) control unit 50, a detection unit 51, a derivation unit 52, a determination unit 53, a generation unit 54, and a display control unit 55.

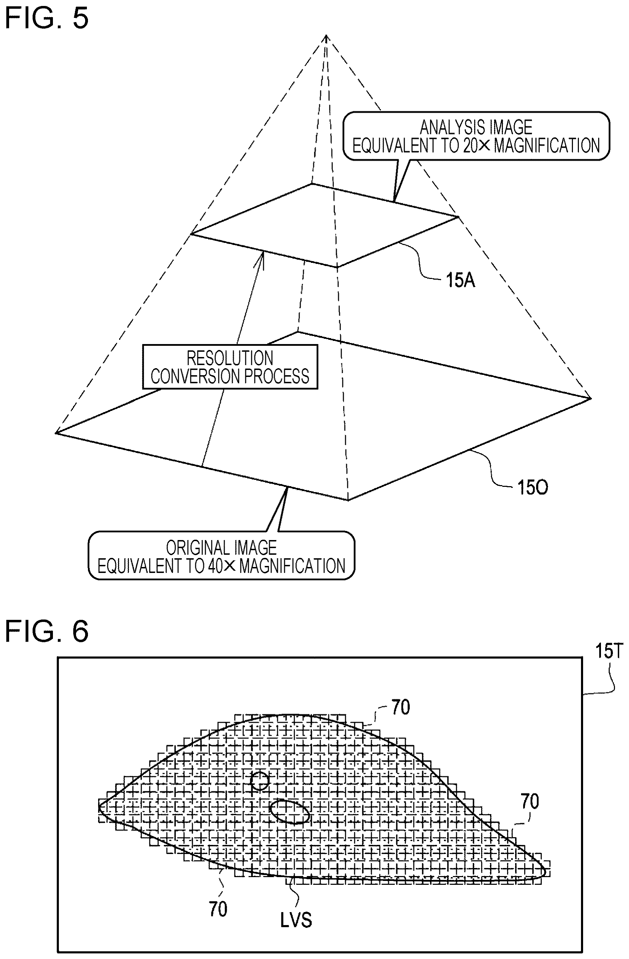

The RW control unit 50 controls storing of various data in the storage 30 and reading of various data from the storage 30. For example, the RW control unit 50 stores a specimen image 15 received from the image capturing device 19 in the storage 30. At this time, as illustrated in Fig. 5 as an example, the RW control unit 50 performs a resolution conversion process on an original specimen image 15 (hereinafter referred to as an original image 15O) received from the image capturing device 19 to generate a specimen image 15 for analysis (hereinafter referred to as an analysis image 15A). The original image 15O has a resolution equivalent to 40× magnification, and the analysis image 15A has a resolution equivalent to 20× magnification. That is, the resolution of the analysis image 15A is half the resolution of the original image 15O. Thus, the load of an analysis process of the analysis image 15A is smaller than that of the original image 15O. The RW control unit 50 stores the original image 15O and the analysis image 15A in the storage 30 in association with each other.

The RW control unit 50 obtains the analysis image 15A designated by the user U via the input device 12 by reading the analysis image 15A from the storage 30. The RW control unit 50 outputs the read analysis image 15A to the detection unit 51, the generation unit 54, and the display control unit 55. The analysis image 15A output from the RW control unit 50 to the detection unit 51 and so forth is a target for detecting whether a morphological abnormality has occurred in the liver specimen LVS. Hereinafter, the analysis image 15A serving as a target for detecting whether a morphological abnormality has occurred in the liver specimen LVS will be referred to as a target specimen image 15T (see Fig. 6 and so forth). A morphological abnormality is a lesion that is not observed in a normal liver specimen LVS, for example, hyperplasia, infiltration, stasis, inflammation, tumor, canceration, proliferation, bleeding, glycogen reduction, or the like.

The RW control unit 50 reads the feature quantity extractor 41 and the detection reference information 42 from the storage 30 and outputs the read feature quantity extractor 41 and detection reference information 42 to the detection unit 51. The RW control unit 50 also reads the determination threshold value 43 from the storage 30 and outputs the read determination threshold value 43 to the determination unit 53. The RW control unit 50 further reads the generation reference information 44 from the storage 30 and outputs the read generation reference information 44 to the generation unit 54.

The detection unit 51 uses the feature quantity extractor 41 and the detection reference information 42 to detect, from among portions of the target specimen image 15T, one or more estimated morphological abnormality portions where a morphological abnormality is estimated to have occurred. The detection unit 51 outputs a detection result 60 of each of the one or more estimated morphological abnormality portions to the derivation unit 52.

The derivation unit 52 derives determination reference information 61 from the detection result 60. The derivation unit 52 outputs the determination reference information 61 to the determination unit 53. Although not illustrated, the derivation unit 52 also outputs the determination reference information 61 serving as an analysis result to the display control unit 55.

The determination unit 53 determines, based on the determination threshold value 43 and the determination reference information 61, whether estimated morphological abnormality portions have been overdetected and whether estimated morphological abnormality portions have been underdetected. The determination unit 53 outputs, to the generation unit 54, a determination result 62 indicating whether estimated morphological abnormality portions have been overdetected and whether estimated morphological abnormality portions have been underdetected. "Estimated morphological abnormality portions have been overdetected" means that a larger number of estimated morphological abnormality portions than the number of true morphological abnormality portions have been detected. "Estimated morphological abnormality portions have been underdetected" means that a smaller number of estimated morphological abnormality portions than the number of true morphological abnormality portions have been detected.

The generation unit 54 generates cause identification reference information 63, based on the target specimen image 15T, the generation reference information 44, and the determination result 62. The generation unit 54 outputs the cause identification reference information 63 to the display control unit 55.

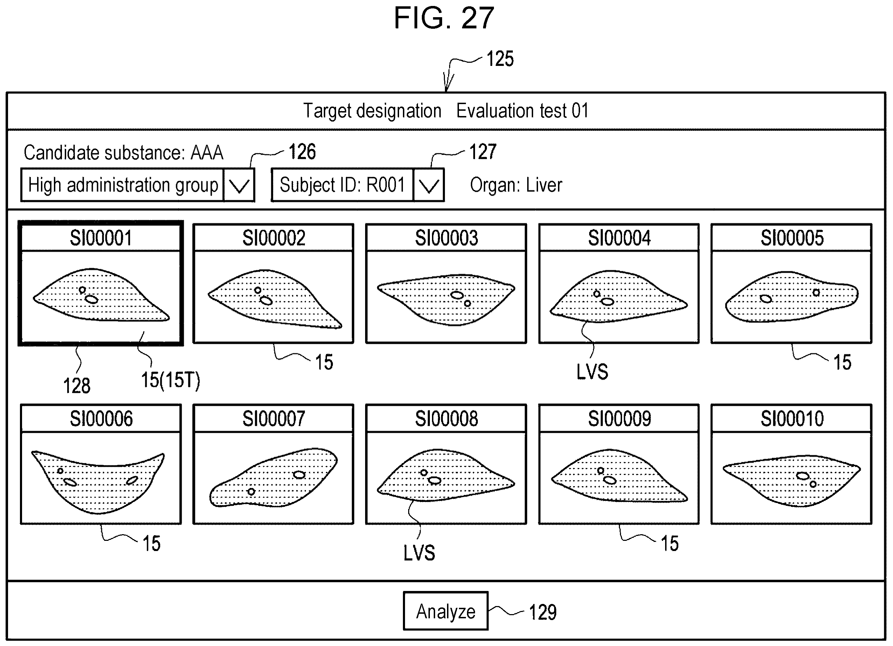

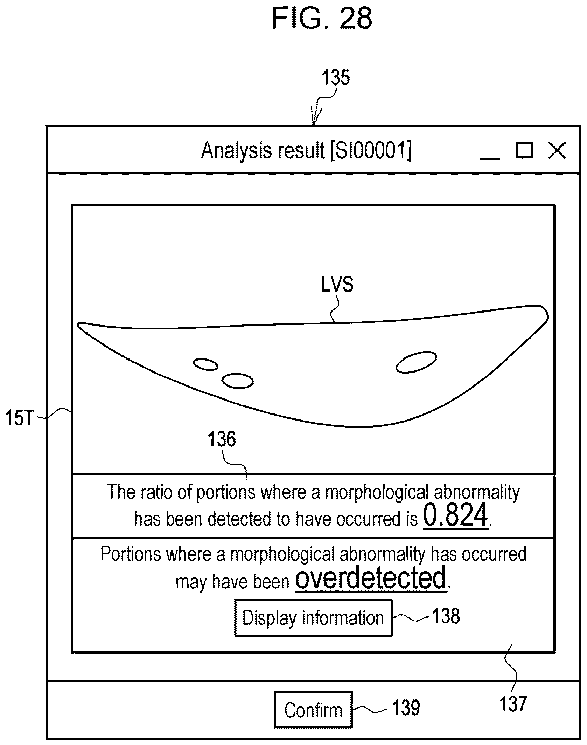

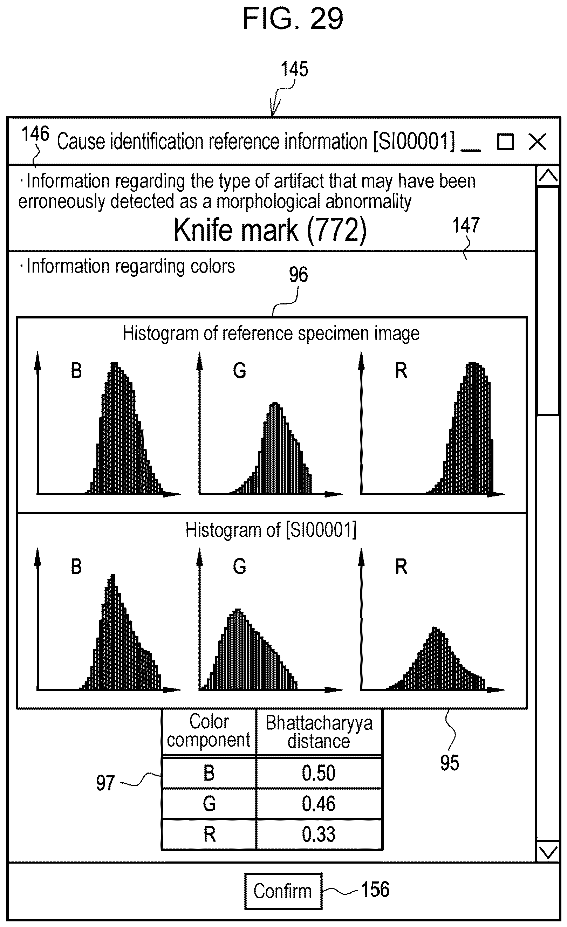

The display control unit 55 performs control to display various screens on the display 11. The various screens include a target designation screen 125 (see Fig. 27) for designating a target specimen image 15T, an analysis result display screen 135 (see Fig. 28), and an information display screen 145 (see Fig. 29 to Fig. 31) for displaying the cause identification reference information 63. The CPU 32 includes constructed therein an instruction receiving unit or the like that receives various operation instructions from the input device 12, in addition to the processing units 50 to 55.

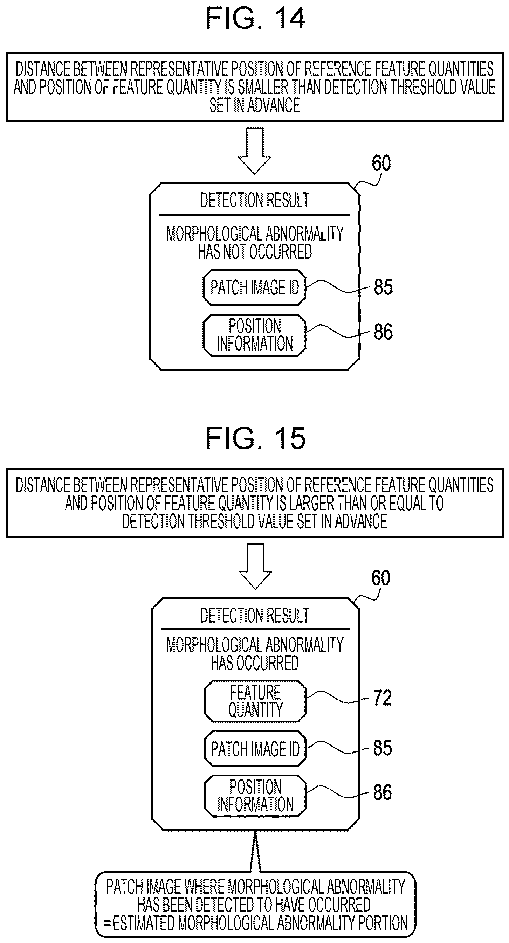

As illustrated in Fig. 6 as an example, the detection unit 51 recognizes the liver specimen LVS in the target specimen image 15T by using a known image recognition technique, and divides the recognized liver specimen LVS into multiple patch images 70. The patch images 70 each have a size that is set in advance and that can be handled by the feature quantity extractor 41. The patch images 70 each have a size that covers not only the portion of a morphological abnormality but also the peripheral region thereof. The detection unit 51 assigns patch image ID 85 (see Fig. 14 and so forth) to each patch image 70. The detection unit 51 associates the patch image ID 85 with information indicating the position of the patch image 70 in the target specimen image 15T, that is, position information 86 (see Fig. 14 and so forth) of the patch image 70. The patch images 70 are an example of "portions of a specimen image" according to the technology of the present disclosure. In Fig. 6, adjacent patch images 70 do not have an overlapping region, but the adjacent patch images 70 may partially overlap each other.

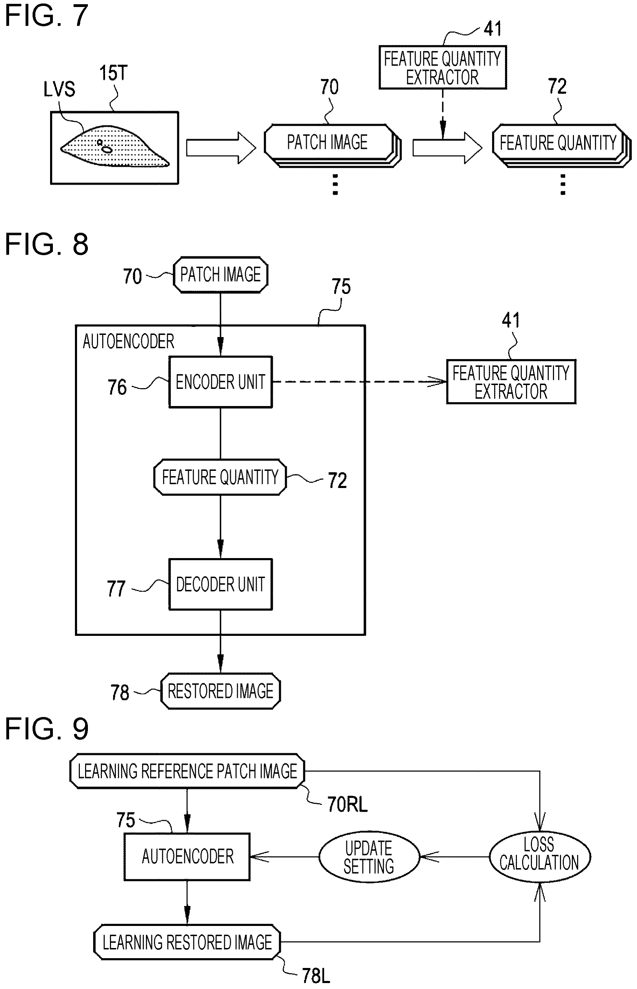

As illustrated in Fig. 7 as an example, the detection unit 51 uses the feature quantity extractor 41 to extract a feature quantity 72 of each of the multiple patch images 70 obtained by dividing the target specimen image 15T. Thus, the number of feature quantities 72 is the same as the number of patch images 70.

As illustrated in Fig. 8 as an example, an encoder unit 76 of an autoencoder 75 is diverted to be used as the feature quantity extractor 41. The autoencoder 75 has a decoder unit 77 in addition to the encoder unit 76. The encoder unit 76 receives a patch image 70. The encoder unit 76 converts the patch image 70 into a feature quantity 72. The encoder unit 76 transfers the feature quantity 72 to the decoder unit 77. The decoder unit 77 generates a restored image 78 of the patch image 70 from the feature quantity 72.

As is known, the encoder unit 76 has a convolutional layer that performs a convolution process using a filter, a pooling layer that performs a pooling process such as a maximum value pooling process, and so forth. The same applies to the decoder unit 77. The encoder unit 76 repeats multiple times a convolution process using the convolutional layer and a pooling process using the pooling layer on the patch image 70 input thereto, thereby extracting the feature quantity 72. The extracted feature quantity 72 represents features of the shape and texture of the liver specimen LVS in the patch image 70.

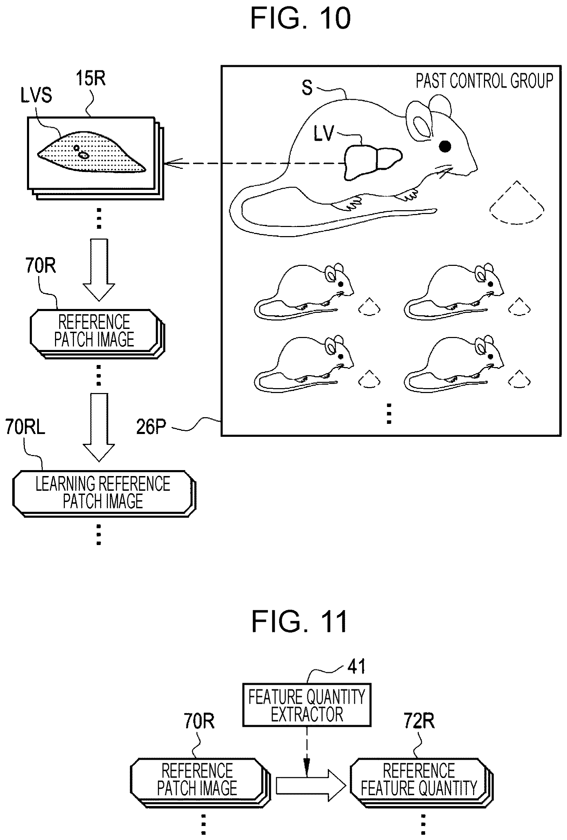

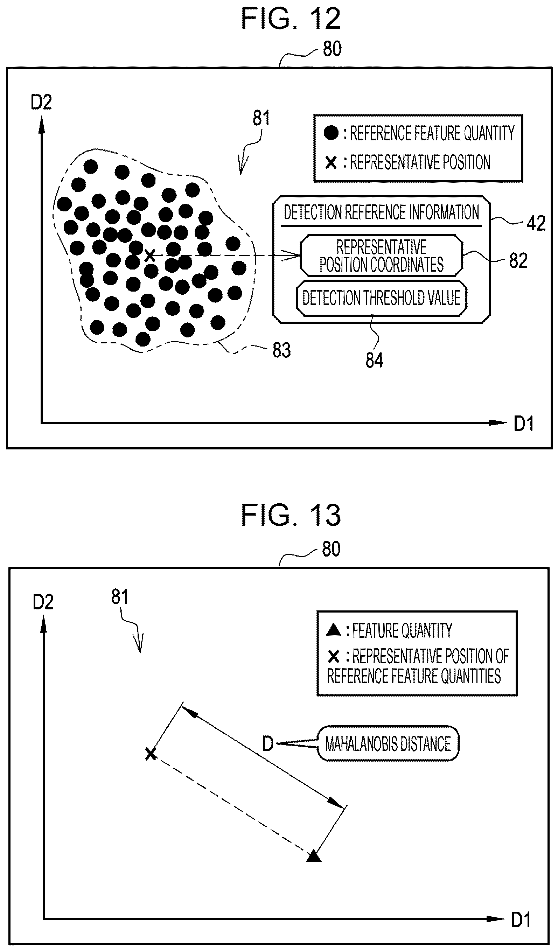

The feature quantity 72 is a set of multiple numerical values. That is, the feature quantity 72 is multidimensional data. The number of dimensions of the feature quantity 72 is, for example, 512, 1024, 2048, or the like. The feature quantity 72 and a reference feature quantity 72R (see Fig. 11) described below have the same number of dimensions, and can be compared in the same feature quantity space 81 (see Fig. 12 and so forth).

As illustrated in Fig. 9 as an example, the autoencoder 75 receives a learning reference patch image 70RL and learns in a learning phase before the encoder unit 76 is diverted to be used as the feature quantity extractor 41. The autoencoder 75 outputs a learning restored image 78L in response to receiving the learning reference patch image 70RL. Based on the learning reference patch image 70RL and the learning restored image 78L, loss calculation of the autoencoder 75 using a loss function is performed. Subsequently, update setting of various coefficients (a coefficient of a filter of the convolutional layer and so forth) of the autoencoder 75 is performed in accordance with the result of the loss calculation, and the autoencoder 75 is updated in accordance with the update setting.

In the learning phase of the autoencoder 75, the above-described series of processes including input of the learning reference patch image 70RL to the autoencoder 75, output of the learning restored image 78L from the autoencoder 75, loss calculation, update setting, and update of the autoencoder 75, is repeatedly performed while the learning reference patch image 70RL is replaced. The repetition of the above-described series of processes is terminated when the restoration accuracy from the learning reference patch image 70RL to the learning restored image 78L has reached a predetermined set level. The encoder unit 76 of the autoencoder 75 whose restoration accuracy has reached the set level is stored as the feature quantity extractor 41 in the storage 30 of the drug discovery support device 10. The learning may be terminated when the above-described series of processes has been repeated a set number of times regardless of the restoration accuracy from the learning reference patch image 70RL to the learning restored image 78L.

Such learning of the autoencoder 75 may be performed by the drug discovery support device 10 or by a device different from the drug discovery support device 10. In the latter case, the feature quantity extractor 41 is transmitted from the different device to the drug discovery support device 10, and the feature quantity extractor 41 is stored in the storage 30 by the RW control unit 50.

As illustrated in Fig. 10 as an example, learning reference patch images 70RL are supplied from multiple reference patch images 70R obtained by dividing a reference specimen image 15R. The reference specimen image 15R is an image of the liver specimen LVS of the subject S in a past control group 26P. The past control group 26P is constituted by multiple subjects S to which a candidate substance was not administered in a past evaluation test. Thus, the number of subjects S constituting the past control group 26P is significantly larger than the number of subjects S constituting the administration group 25 and the control group 26 and is, for example, about several hundred to several thousand. Like the specimen images 15, multiple reference specimen images 15R are obtained from a single subject S. Thus, the number of reference specimen images 15R obtained from the past control group 26P is equal to the number obtained by multiplying the number of reference specimen images 15R obtained from a single subject S by the number of subjects S. The liver specimen LVS of the subject S in the past control group 26P is an example of "a tissue specimen regarded to be normal" according to the technology of the present disclosure. In addition to the specimen image 15 of the liver specimen LVS of the subject S in the past control group 26P, the specimen image 15 of the liver specimen LVS determined to be normal by an expert such as a pathologist in a past administration group constituted by multiple subjects S to which a candidate substance was administered in a past evaluation test may be employed as the reference specimen image 15R.

Next, the composition of the detection reference information 42 will be described. First, as illustrated in Fig. 11 as an example, the feature quantity extractor 41 is used to extract multiple reference feature quantities 72R from multiple reference patch images 70R that are based on all of multiple reference specimen images 15R.

A graph 80 illustrated in Fig. 12 as an example is obtained by plotting the multiple reference feature quantities 72R extracted in Fig. 11 in the feature quantity space 81. The detection reference information 42 includes the coordinates of a representative position of the reference feature quantities 72R indicated by a cross mark (hereinafter referred to as representative position coordinates) 82 in the feature quantity space 81. The representative position is, for example, a center point or an average point of a distribution 83 of the reference feature quantities 72R. The detection reference information 42 also includes a detection threshold value 84. In Fig. 12, for convenience of description, the dimensions of the feature quantity space 81 are two dimensions having a D1 axis and a D2 axis, but the actual dimensions of the feature quantity space 81 are 512 dimensions or the like, as described above. Also in Fig. 13 and so forth, for convenience of description, the dimensions of the feature quantity space 81 are represented by two dimensions.

As in the learning of the autoencoder 75, the representative position coordinates 82 of the detection reference information 42 may be derived by the drug discovery support device 10 or may be derived by a device different from the drug discovery support device 10. In the latter case, the representative position coordinates 82 are transmitted from the different device to the drug discovery support device 10, and the representative position coordinates 82 are stored in the storage 30 by the RW control unit 50.

As illustrated in Fig. 13 as an example, the detection unit 51 calculates a distance D in the feature quantity space 81 between the representative position of the reference feature quantities 72R represented by the representative position coordinates 82 in the detection reference information 42 and the position of the feature quantity 72. The detection unit 51 calculates distances D for the multiple feature quantities 72 extracted for the multiple patch images 70 obtained by dividing a single target specimen image 15T. The distance D is a Mahalanobis distance. The distance D indicates the degree of deviation of the feature quantity 72 from the reference feature quantity 72R, more specifically, the degree of deviation of the liver specimen LVS in the patch image 70 of the target specimen image 15T from the liver specimen LVS regarded to be normal. That is, as the distance D increases, the liver specimen LVS in the patch image 70 deviates more from the liver specimen LVS regarded to be normal. Thus, as the distance D increases, the probability that a morphological abnormality has occurred in the liver specimen LVS in the patch image 70 increases.

As the distance D, any one of an average value, a median value, and a maximum value of the Euclidean distances between the positions of k-nearest neighbor samples of the distribution 83 of the reference feature quantities 72R and the position of the feature quantity 72 may be calculated. Alternatively, instead of the distance D, a value obtained by subtracting, from 1.0, the cosine similarity between a vector representing the representative position of the reference feature quantities 72R and a vector representing the position of the feature quantity 72 may be calculated. The cosine similarity takes a value between -1.0 and 1.0. As the value increases, the similarity of the orientations of the vectors increases. Furthermore, instead of the distance D, a likelihood function such as a negative logarithmic likelihood may be calculated as the degree of deviation.

As illustrated in Fig. 14 and Fig. 15 as an example, the detection unit 51 compares the calculated distance D with the detection threshold value 84. As illustrated in Fig. 14, when the distance D is smaller than the detection threshold value 84, the detection unit 51 detects that a morphological abnormality has not occurred in the liver specimen LVS in the patch image 70. The detection unit 51 outputs the detection result 60 indicating that a morphological abnormality has not occurred in the liver specimen LVS in the patch image 70. The detection result 60 in this case includes the patch image ID 85 and the position information 86.

On the other hand, as illustrated in Fig. 15, when the distance D is larger than or equal to the detection threshold value 84, the detection unit 51 detects that a morphological abnormality has occurred in the liver specimen LVS in the patch image 70. The detection unit 51 outputs the detection result 60 indicating that a morphological abnormality has occurred in the liver specimen LVS in the patch image 70. In this case, the detection result 60 includes the feature quantity 72 in addition to the patch image ID 85 and the position information 86. The portion of the patch image 70 where a morphological abnormality has been detected to have occurred in the liver specimen LVS in this way corresponds to "an estimated morphological abnormality portion" according to the technology of the present disclosure. The detection threshold value 84 may be common among the high administration group 25H, the medium administration group 25M, the low administration group 25L, and the control group 26, or may be different among these groups. Furthermore, instead of using the distance D, the cosine similarity or the likelihood function described above may be compared with the detection threshold value 84 to detect whether a morphological abnormality has occurred in the liver specimen LVS in the patch image 70.

As illustrated in Fig. 10 and Fig. 11, the reference feature quantities 72R are feature quantities extracted from the reference patch images 70R obtained by dividing the reference specimen image 15R of the liver specimen LVS of the subject S in the past control group 26P. Because the subject S in the past control group 26P is the subject S to which a candidate substance was not administered, at least a morphological abnormality resulting from the toxicity of the candidate substance has not occurred in the liver specimen LVS in the reference specimen image 15R. Accordingly, the representative position of the reference feature quantities 72R is regarded as the representative position of the feature quantity of the specimen image 15 of a normal liver specimen LVS. Thus, the distance D between the representative position of the reference feature quantities 72R and the position of the feature quantity 72 serves as an index indicating the degree of deviation of the liver specimen LVS in the patch image 70 from the normal liver specimen LVS, as described above. Thus, as illustrated in Fig. 14, for the patch image 70 in which the distance D is smaller than the detection threshold value 84, the detection unit 51 determines that the liver specimen LVS in the patch image 70 does not deviate from the normal liver specimen LVS and detects that a morphological abnormality has not occurred. On the other hand, as illustrated in Fig. 15, for the patch image 70 in which the distance D is larger than or equal to the detection threshold value 84, the detection unit 51 determines that the liver specimen LVS in the patch image 70 deviates from the normal liver specimen LVS and detects that a morphological abnormality has occurred.

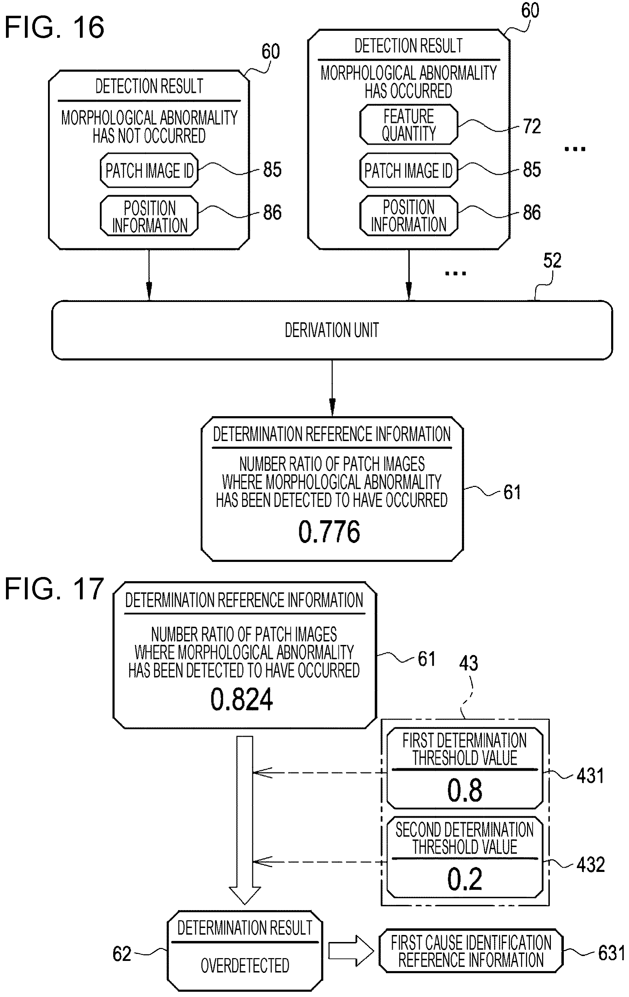

As illustrated in Fig. 16 as an example, the detection results 60 of all the patch images 70 are input to the derivation unit 52. The derivation unit 52 counts the number of detection results 60 indicating that a morphological abnormality has occurred in the liver specimen LVS in the patch image 70, that is, the number of estimated morphological abnormality portions. The counted number is divided by the total number of patch images 70 to calculate a number ratio of the patch images 70 where a morphological abnormality has been detected to have occurred, that is, a number ratio of estimated morphological abnormality portions. The derivation unit 52 outputs the calculated number ratio as the determination reference information 61. The number ratio is an example of "a numerical value related to the number of estimated morphological abnormality portions" according to the technology of the present disclosure. Instead of the number ratio, the number of estimated morphological abnormality portions may be output as the determination reference information 61.

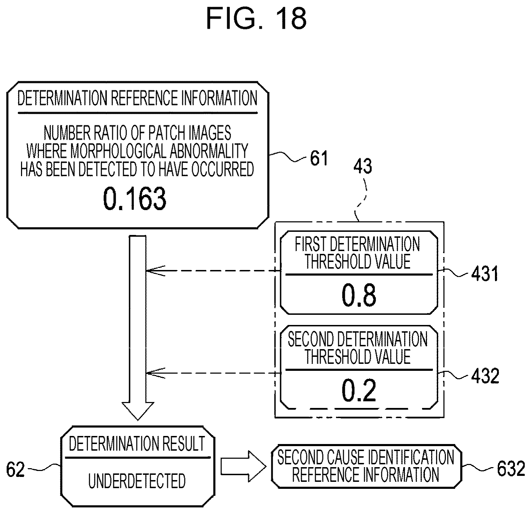

As illustrated in Fig. 17 and Fig. 18 as an example, the determination threshold value 43 includes a first determination threshold value 431 and a second determination threshold value 432. The determination unit 53 compares the number ratio in the determination reference information 61 with the first determination threshold value 431 and the second determination threshold value 432.

As illustrated in Fig. 17, when the number ratio in the determination reference information 61 is larger than or equal to the first determination threshold value 431, the determination unit 53 determines that the patch images 70 where a morphological abnormality has been detected to have occurred, that is, estimated morphological abnormality portions, have been overdetected, and outputs the determination result 62 indicating the fact. In this case, the generation unit 54 generates, as the cause identification reference information 63, first cause identification reference information 631 contributing to identifying the cause of the estimated morphological abnormality portions having been overdetected.

On the other hand, as illustrated in Fig. 18, when the number ratio in the determination reference information 61 is smaller than or equal to the second determination threshold value 432, the determination unit 53 determines that the patch images 70 where a morphological abnormality has been detected to have occurred, that is, estimated morphological abnormality portions, have been underdetected, and outputs the determination result 62 indicating the fact. In this case, the generation unit 54 generates, as the cause identification reference information 63, second cause identification reference information 632 contributing to identifying the cause of the estimated morphological abnormality portions having been underdetected.

Although not illustrated, when the number ratio in the determination reference information 61 is neither larger than or equal to the first determination threshold value 431 nor smaller than or equal to the second determination threshold value 432, that is, when the number ratio in the determination reference information 61 is larger than the second determination threshold value 432 and smaller than the first determination threshold value 431, the determination unit 53 outputs the determination result 62 indicating that the number of detected patch images 70 where a morphological abnormality has been detected to have occurred, that is, the number of detected estimated morphological abnormality portions, is appropriate. In this case, the generation unit 54 does not generate the cause identification reference information 63. The first determination threshold value 431 and the second determination threshold value 432 may be common among the high administration group 25H, the medium administration group 25M, the low administration group 25L, and the control group 26, or may be different among these groups.

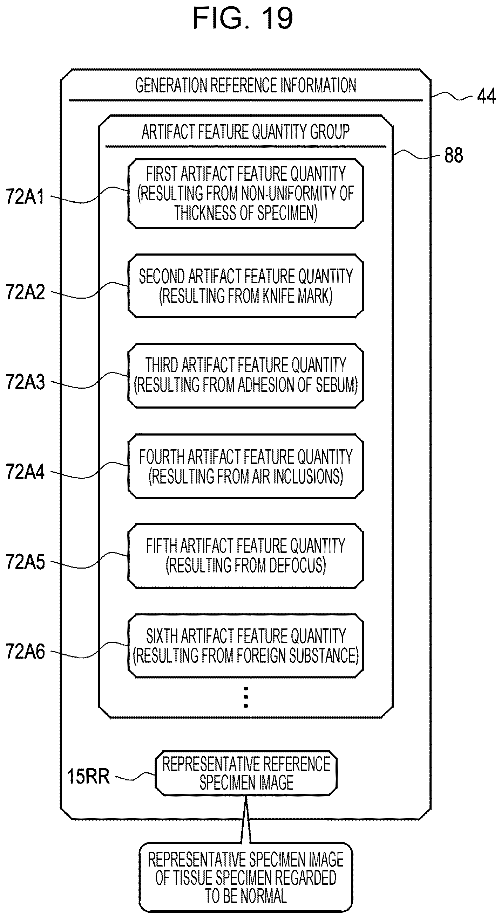

As illustrated in Fig. 19 as an example, the generation reference information 44 includes an artifact feature quantity group 88 and a representative reference specimen image 15RR. The artifact feature quantity group 88 is a set of feature quantities of multiple types of artifacts that may be erroneously detected as an estimated morphological abnormality portion (hereinafter referred to as artifact feature quantities) 72A. The representative reference specimen image 15RR is an image representing a tint, such as the degree of staining with a hematoxylin-eosin stain, among multiple reference specimen images 15R.

An artifact that may be erroneously detected as an estimated morphological abnormality portion occurs at the time of preparing the specimen slide 18 and at the time of capturing the specimen image 15 by the image capturing device 19. The artifact includes a first artifact resulting from non-uniformity of the thickness of a specimen (here, the liver specimen LVS), a second artifact resulting from a knife mark generated when the specimen is collected, a third artifact resulting from adhesion of sebum to the specimen, and the like. The artifact also includes a fourth artifact resulting from air inclusions under the coverslip 17, a fifth artifact resulting from defocus at the time of capturing by the image capturing device 19, a sixth artifact resulting from a foreign substance such as dust, and the like.

The artifact feature quantities 72A have a first artifact feature quantity 72A1, which is the feature quantity 72 of the first artifact, a second artifact feature quantity 72A2, which is the feature quantity 72 of the second artifact, a third artifact feature quantity 72A3, which is the feature quantity 72 of the third artifact, and the like. The artifact feature quantities 72A also have a fourth artifact feature quantity 72A4, which is the feature quantity 72 of the fourth artifact, a fifth artifact feature quantity 72A5, which is the feature quantity 72 of the fifth artifact, a sixth artifact feature quantity 72A6, which is the feature quantity 72 of the sixth artifact, and the like.

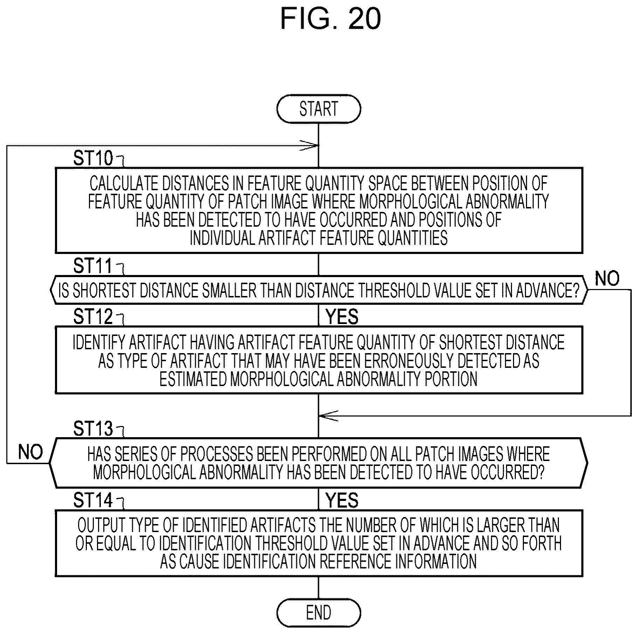

The generation unit 54 generates the cause identification reference information 63 by the procedure illustrated in Fig. 20 to Fig. 22 as an example. First, the generation unit 54 calculates the distances D in the feature quantity space 81 between the position of the feature quantity 72 of the patch image 70 where a morphological abnormality has been detected to have occurred and the positions of the individual artifact feature quantities 72A (step ST10).

The generation unit 54 compares a shortest distance SD among the calculated distances D with a distance threshold value set in advance (step ST11). When the shortest distance SD is smaller than the distance threshold value (YES in step ST11), the generation unit 54 identifies the artifact having the artifact feature quantity 72A of the shortest distance SD as the type of artifact that may have been erroneously detected as an estimated morphological abnormality portion (step ST12). On the other hand, when the shortest distance SD is larger than or equal to the distance threshold value (NO in step ST11), the process proceeds to step ST13.

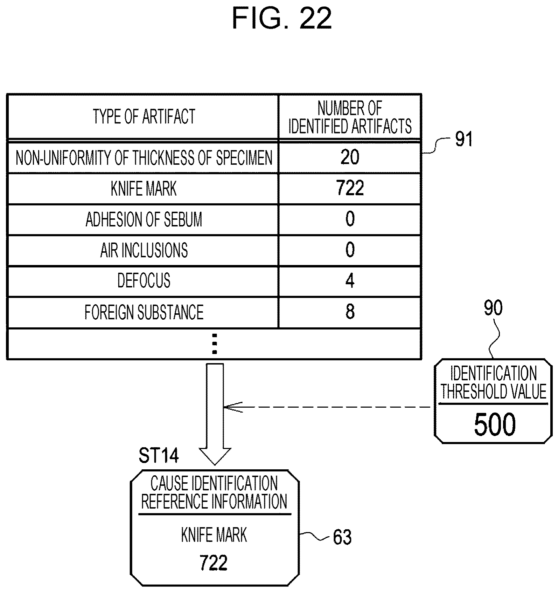

The generation unit 54 continues the series of processes from step ST10 to step ST12 until the series of processes has been performed on all the patch images 70 where a morphological abnormality has been detected to have occurred (NO in step ST13). When the series of processes from step ST10 to step ST12 has been performed on all the patch images 70 where a morphological abnormality has been detected to have occurred (YES in step ST13), the generation unit 54 outputs, as the cause identification reference information 63 (the first cause identification reference information 631 and the second cause identification reference information 632), the type of identified artifacts the number of which is larger than or equal to an identification threshold value 90 set in advance (see Fig. 22) and the number of the identified artifacts (step ST14). The type of artifacts and the number of identified artifacts are an example of "information regarding the type" according to the technology of the present disclosure.

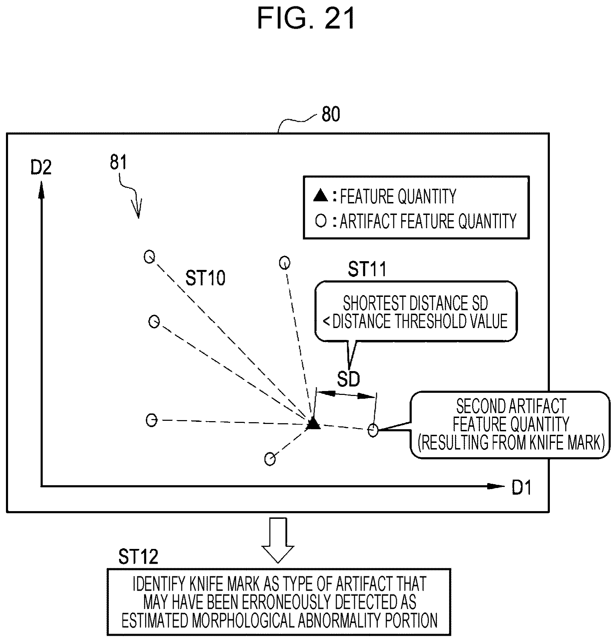

Fig. 21 illustrates a case where the distance D between the position of the feature quantity 72 of the patch image 70 where a morphological abnormality has been detected to have occurred and the position of the second artifact feature quantity 72A2 is the shortest distance SD and the shortest distance SD is smaller than the distance threshold value. The second artifact feature quantity 72A2 is the feature quantity of the second artifact resulting from a knife mark. Thus, in this case, the generation unit 54 identifies the knife mark as the type of artifact that may have been erroneously detected as an estimated morphological abnormality portion.

Fig. 22 illustrates, as illustrated in a table 91, a case where the number of identified artifacts resulting from non-uniformity of the thickness of a specimen is 20, the number of identified artifacts resulting from a knife mark is 722, the number of identified artifacts resulting from adhesion of sebum and air inclusions is 0, ···, and the identification threshold value 90 is 500. In this case, the generation unit 54 outputs the cause identification reference information 63 including knife mark and the number of identified artifacts 722 thereof. The identification threshold value 90 may be common among the high administration group 25H, the medium administration group 25M, the low administration group 25L, and the control group 26, or may be different among these groups.

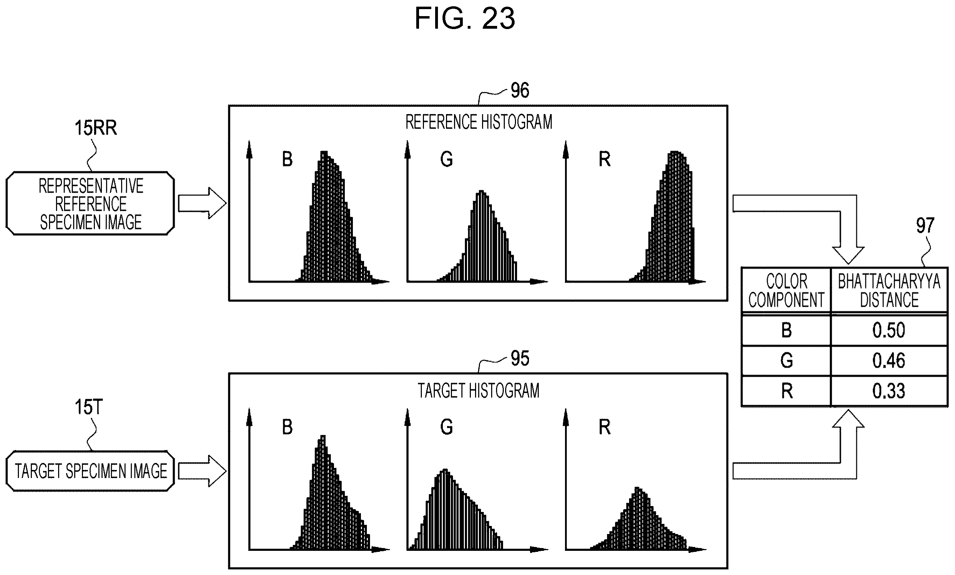

As illustrated in Fig. 23 as an example, the generation unit 54 generates, from the target specimen image 15T, a histogram of pixel values of pixels of individual colors of blue (B), green (G), and red (R) (hereinafter referred to as a target histogram) 95. The generation unit 54 also generates, from the representative reference specimen image 15RR, a histogram of pixel values of pixels of the individual colors (hereinafter referred to as a reference histogram) 96. Furthermore, as illustrated in a table 97, the generation unit 54 calculates a Bhattacharyya distance between B distributions, a Bhattacharyya distance between G distributions, and a Bhattacharyya distance between R distributions in the target histogram 95 and the reference histogram 96. The Bhattacharyya distance has a value closer to 0 as the similarity between the distributions increases. The generation unit 54 outputs, as the cause identification reference information 63 (the first cause identification reference information 631 and the second cause identification reference information 632), the target histogram 95, the reference histogram 96, and the Bhattacharyya distances. The target histogram 95 is an example of "information regarding a color of a specimen image" according to the technology of the present disclosure. The reference histogram 96 is an example of "information regarding a color of a reference specimen image" according to the technology of the present disclosure. The Bhattacharyya distance is an example of "information regarding a color of a specimen image" and "information regarding a color of a reference specimen image" according to the technology of the present disclosure. Instead of the representative reference specimen image 15RR, the reference histogram 96 may be stored as the generation reference information 44.

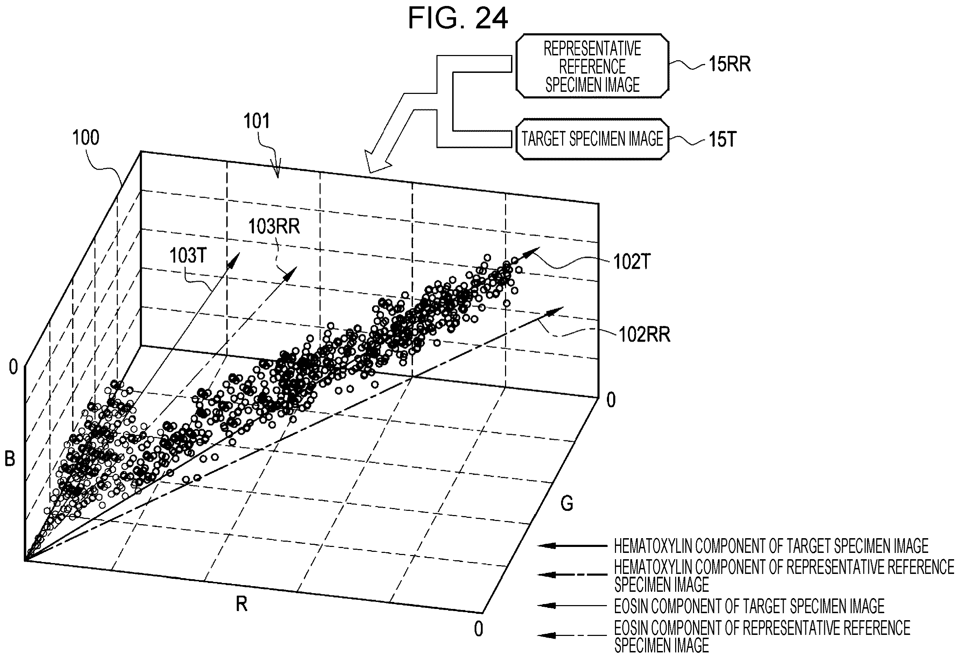

As illustrated in Fig. 24 as an example, the generation unit 54 extracts a hematoxylin component 102T and an eosin component 103T of the target specimen image 15T from a distribution of the pixel values of the pixels of the target specimen image 15T in an RGB space 101 indicated by a graph 100. The generation unit 54 also extracts a hematoxylin component 102RR and an eosin component 103RR of the representative reference specimen image 15RR.

According to the hematoxylin component 102T and the eosin component 103T of the target specimen image 15T and the hematoxylin component 102RR and the eosin component 103RR of the representative reference specimen image 15RR, it is possible to visualize the difference between a staining condition for the liver specimen LVS in the target specimen image 15T and a staining condition for the liver specimen LVS in the representative reference specimen image 15RR. The generation unit 54 outputs, as the cause identification reference information 63 (the first cause identification reference information 631 and the second cause identification reference information 632), the hematoxylin component 102T and the eosin component 103T of the target specimen image 15T and the hematoxylin component 102RR and the eosin component 103RR of the representative reference specimen image 15RR.

The hematoxylin component 102T and the eosin component 103T of the target specimen image 15T are an example of "information regarding a color of a specimen image" according to the technology of the present disclosure. The hematoxylin component 102RR and the eosin component 103RR of the representative reference specimen image 15RR are an example of "information regarding a color of a reference specimen image" according to the technology of the present disclosure. Instead of the representative reference specimen image 15RR, the hematoxylin component 102RR and the eosin component 103RR of the representative reference specimen image 15RR may be stored as the generation reference information 44.

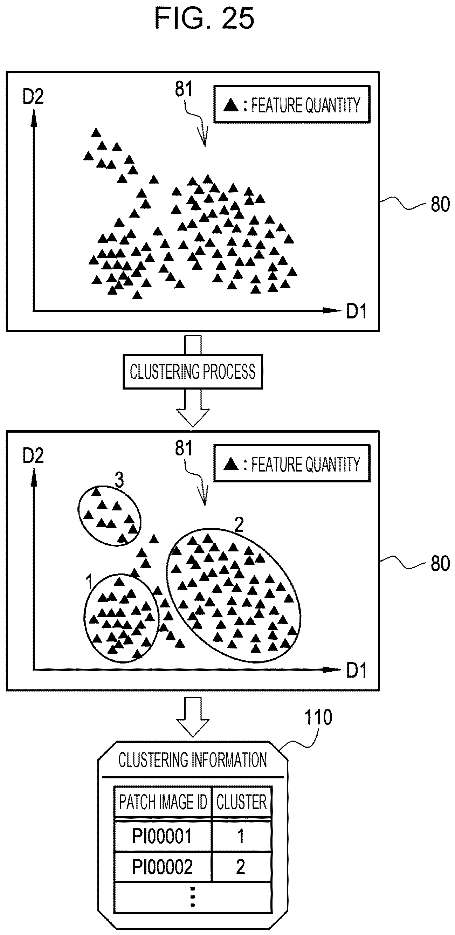

As illustrated in Fig. 25 as an example, in response to the determination unit 53 determining that estimated morphological abnormality portions have been overdetected, the generation unit 54 performs, on the feature quantities 72 of the patch images 70 where a morphological abnormality has been detected to have occurred, a clustering process of defining a cluster to which each of the feature quantities 72 belongs. Fig. 25 illustrates an example in which the feature quantities 72 are clustered into three clusters of a cluster 1, a cluster 2, and a cluster 3. As illustrated, some of the feature quantities 72 do not belong to any of the clusters.

The generation unit 54 generates clustering information 110. The clustering information 110 is information in which the clusters to which the individual patch images 70 belong are registered based on the patch image ID 85. The patch image 70 whose feature quantity 72 does not belong to any cluster is not registered in the clustering information 110.

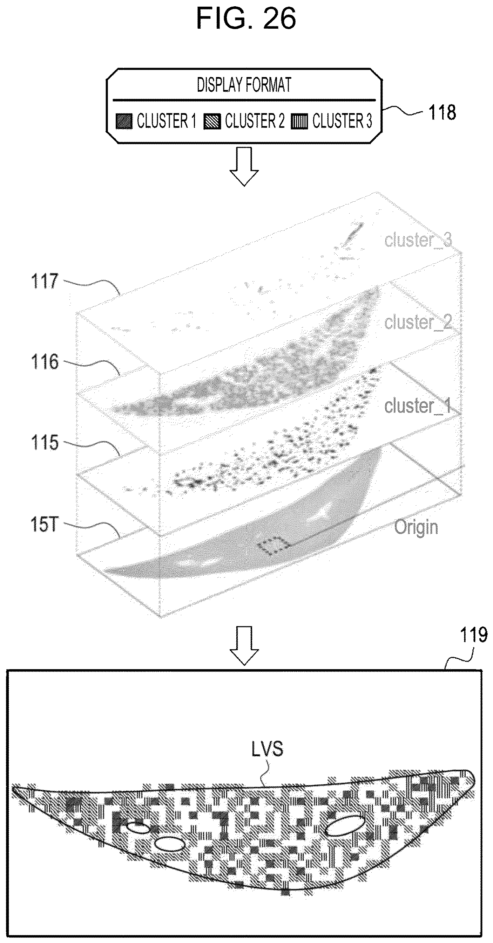

As illustrated in Fig. 26 as an example, the generation unit 54 processes the target specimen image 15T in accordance with the clustering information 110, thereby generating cluster images 115, 116, and 117 as the first cause identification reference information 631. The cluster image 115 is an image corresponding to the cluster 1. The cluster image 116 is an image corresponding to the cluster 2. The cluster image 117 is an image corresponding to the cluster 3.

The generation unit 54 generates the cluster images 115 to 117 in accordance with a display format 118 set in advance for each cluster. In the display format 118, for example, the cluster 1 is displayed in indigo, the cluster 2 is displayed in yellowish green, and the cluster 3 is displayed in gray. The generation unit 54 generates the cluster image 115 by filling with indigo the positions of the patch images 70 having the patch image ID 85 for which the cluster 1 is registered in the clustering information 110 (known from the position information 86) in the target specimen image 15T. Similarly, the generation unit 54 generates the cluster image 116 by filling with yellowish green the positions of the patch images 70 having the patch image ID 85 for which the cluster 2 is registered in the clustering information 110 in the target specimen image 15T. Furthermore, the generation unit 54 generates the cluster image 117 by filling with gray the positions of the patch images 70 having the patch image ID 85 for which the cluster 3 is registered in the clustering information 110 in the target specimen image 15T. With use of the different display colors, the cluster images 115 to 117 enable the clusters 1 to 3 to be identified. The display format 118 may be configured such that the user U is able to freely change the setting.

The generation unit 54 generates a superimposed image 119 in which at least one of the cluster images 115 to 117 is superimposed on the target specimen image 15T. Fig. 26 illustrates the superimposed image 119 in which all the cluster images 115 to 117 are superimposed on the target specimen image 15T.

As illustrated in Fig. 27 as an example, the display control unit 55 performs control to display the target designation screen 125 on the display 11. The display control unit 55 displays the target designation screen 125 in response to an instruction to display a specimen image 15 being provided by the user U via the input device 12. Multiple specimen images 15 are displayed side by side on the target designation screen 125. The multiple specimen images 15 are specimen images 15 obtained from a single subject S among multiple subjects S constituting any one group selected in a pull-down menu 126 from among the high administration group 25H, the medium administration group 25M, the low administration group 25L, and the control group 26. Fig. 27 illustrates an example in which ten specimen images 15 having specimen image IDs "SI00001" to "SI00010" obtained from the subject S having a subject ID "R001" are displayed side by side. Subject IDs are in a pull-down menu 127, and the subject S whose specimen images 15 are to be displayed on the target designation screen 125 can be switched.

The target designation screen 125 is a screen for designating one target specimen image 15T from among multiple specimen images 15. The target designation screen 125 is provided with one selection frame 128 that is movable between the specimen images 15. An analyze button 129 is provided in a lower part of the target designation screen 125. The user U sets the selection frame 128 to a desired specimen image 15 and then selects the analyze button 129. Accordingly, with the specimen image 15 to which the selection frame 128 is set serving as the target specimen image 15T, the detection of an estimated morphological abnormality portion by the detection unit 51, the derivation of the determination reference information 61 by the derivation unit 52, the determination of whether estimated morphological abnormality portions have been overdetected or underdetected by the determination unit 53, and the generation of the cause identification reference information 63 by the generation unit 54 are performed.

After the above-described processes by the individual processing units have been completed, the display control unit 55 performs control to display the analysis result display screen 135 illustrated in Fig. 28 as an example on the display 11. The target specimen image 15T is displayed on the analysis result display screen 135. A first display region 136 and a second display region 137 are provided below the target specimen image 15T. The number ratio of estimated morphological abnormality portions included in the determination reference information 61 received from the derivation unit 52 is displayed as an analysis result in the first display region 136. In response to the determination unit 53 determining that estimated morphological abnormality portions have been overdetected or underdetected, a message indicating the fact is displayed in the second display region 137. Fig. 28 illustrates a case where the determination unit 53 determines that estimated morphological abnormality portions have been overdetected. Although not illustrated, in response to the determination unit 53 not determining that estimated morphological abnormality portions have been overdetected or underdetected, a message indicating that the number of detected estimated morphological abnormality portions is appropriate is displayed in the second display region 137.

An information display button 138 is provided in the second display region 137. The information display button 138 is a button for displaying the cause identification reference information 63. The display control unit 55 hides the analysis result display screen 135 in response to a confirm button 139 being selected.

Here, in the target specimen image 15T on the analysis result display screen 135, the portion of the patch image 70 where a morphological abnormality has been detected to have occurred may be distinguishably displayed by, for example, filling the portion in red. At this time, as the difference between the distance D and the detection threshold value 84 increases, the depth of the color may increase, or the filling color may be changed in accordance with the type of artifact identified in the generation unit 54.

In response to the information display button 138 being selected, the display control unit 55 performs control to display the information display screen 145 illustrated in Fig. 29 to Fig. 31 as an example on the display 11. The information display screen 145 is provided with a first display region 146, a second display region 147, a third display region 148, and a fourth display region 149. The first display region 146 to the fourth display region 149 can be scroll-displayed.

In the first display region 146, the type of artifacts identified as having a possibility of having been erroneously detected as an estimated morphological abnormality portion, and the number of the identified artifacts are displayed. In the second display region 147, the target histogram 95, the reference histogram 96, and the table 97 of Bhattacharyya distances are displayed.

In the second display region 147, the graph 100 including the hematoxylin component 102T and the eosin component 103T of the target specimen image 15T and the hematoxylin component 102RR and the eosin component 103RR of the representative reference specimen image 15RR, and a legend 150 are displayed.

In the third display region 148, the superimposed image 119 and a legend 151 are displayed. Display switch buttons 152, 153, and 154 are provided below the legend 151. The display switch button 152 is a button for selecting whether to superimpose and display the cluster image 115 on the target specimen image 15T. The display switch button 153 is a button for selecting whether to superimpose and display the cluster image 116 on the target specimen image 15T. The display switch button 154 is a button for selecting whether to superimpose and display the cluster image 117 on the target specimen image 15T. Thus, for example, when all the display switch buttons 152 to 154 are selected as illustrated in the figure, the display control unit 55 displays the superimposed image 119 in which all the cluster images 115 to 117 are superimposed on the target specimen image 15T. In this way, the display control unit 55 superimposes and displays at least one of the multiple cluster images 115 to 117 on the target specimen image 15T. The information display screen 145 is initially displayed in a state where all the display switch buttons 152 to 154 are selected.

In this way, by displaying the first cause identification reference information 631 or the second cause identification reference information 632 in the first display region 146 to the third display region 148, the display control unit 55 presents the first cause identification reference information 631 or the second cause identification reference information 632 to the user U.

In the fourth display region 149, a message 155 prompting the user U to perform an improvement process is displayed. The improvement process includes a suppression process and an exclusion process. The suppression process is a process of suppressing overdetection or underdetection of estimated morphological abnormality portions. The exclusion process is a process of excluding a portion of an artifact that may have been erroneously detected as an estimated morphological abnormality portion from the target of an evaluation test. The exclusion from the target of an evaluation test means excluding the patch image 70 including an artifact that may have been erroneously detected as an estimated morphological abnormality portion from the calculation target of a number ratio.

When there is a type of artifact (a knife mark in this case) identified as having a possibility of having been erroneously detected as an estimated morphological abnormality portion, a message 155A prompting the user U to perform an exclusion process is displayed in the fourth display region 149. When the tint of the target specimen image 15T and the tint of the representative reference specimen image 15RR are different from each other by a tint threshold value set in advance or more, a message 155B prompting the user U to perform a process of correcting the color of the target specimen image 15T as a suppression process is displayed in the fourth display region 149. Here, the tint threshold value is set with respect to the Bhattacharyya distance (for example, 0.5 or the like). Alternatively, the tint threshold value is set with respect to the distance in the RGB space 101 between the hematoxylin component 102T and the eosin component 103T of the target specimen image 15T and the hematoxylin component 102RR and the eosin component 103RR of the representative reference specimen image 15RR. In response to a confirm button 156 being selected, the display control unit 55 hides the information display screen 145.

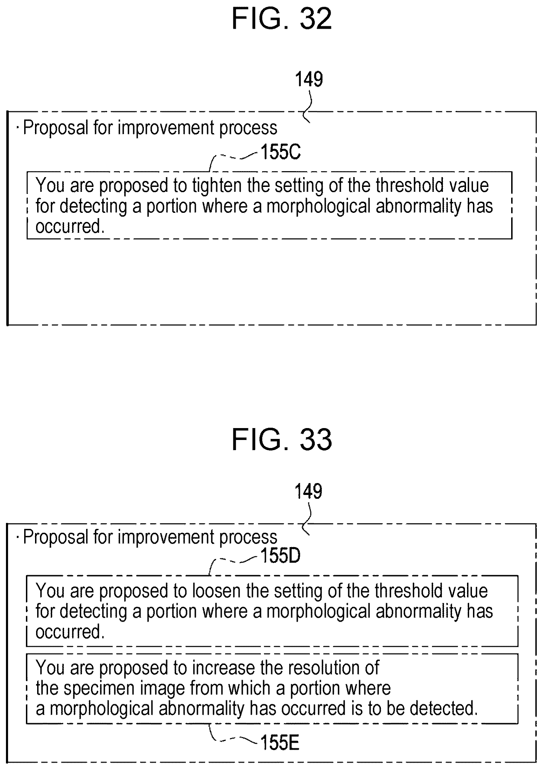

As illustrated in Fig. 32 and Fig. 33 as an example, the message 155 prompting the user U to perform an improvement process has variations. A message 155C illustrated in Fig. 32 is displayed when the determination unit 53 determines that estimated morphological abnormality portions have been overdetected. The message 155C is also displayed when there is not the type of artifact identified as having a possibility of having been erroneously detected as an estimated morphological abnormality portion and the tint of the target specimen image 15T and the tint of the representative reference specimen image 15RR are not different from each other by the tint threshold value or more. The message 155C prompts the user U to perform a process of changing the detection threshold value 84 to be used to detect an estimated morphological abnormality portion. To be more specific, the message 155C prompts the user U to tighten the setting of the detection threshold value 84. To tighten the setting of the detection threshold value 84 means to reset the detection threshold value 84 to a larger value.

Messages 155D and 155E illustrated in Fig. 33 are displayed when the determination unit 53 determines that estimated morphological abnormality portions have been underdetected. The messages 155D and 155E are also displayed when there is not the type of artifact identified as having a possibility of having been erroneously detected as an estimated morphological abnormality portion and the tint of the target specimen image 15T and the tint of the representative reference specimen image 15RR are not different from each other by the tint threshold value or more. Like the message 155C, the message 155D prompts the user U to perform a process of changing the detection threshold value 84 to be used to detect an estimated morphological abnormality portion. However, contrary to the message 155C, the message 155D prompts the user U to loosen the setting of the detection threshold value 84. To loosen the setting of the detection threshold value 84 means to reset the detection threshold value 84 to a smaller value.

The message 155E prompts the user U to perform a process of changing the resolution of the target specimen image 15T. To be more specific, the message 155E prompts the user U to increase the resolution of the target specimen image 15T. To increase the resolution of the target specimen image 15T refers to using the original image 15O instead of the analysis image 15A as the target specimen image 15T.

By displaying the message 155 in the fourth display region 149 in this way, the display control unit 55 proposes a suppression process and/or an exclusion process to the user U.

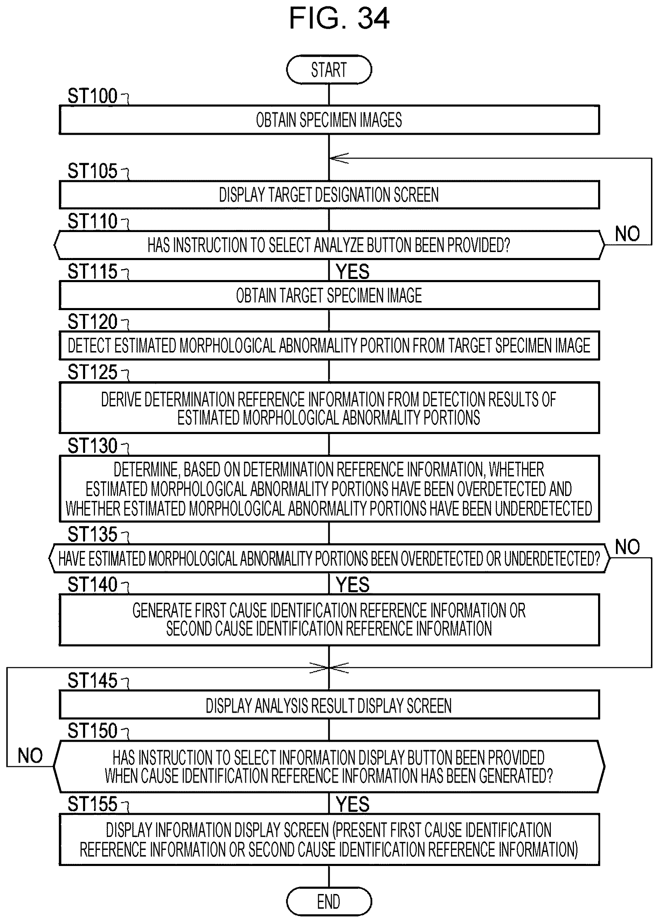

Next, the operation of the above-described configuration will be described with reference to the flowchart illustrated in Fig. 34 as an example. First, upon the operation program 40 being started in the drug discovery support device 10, the CPU 32 of the drug discovery support device 10 functions as the RW control unit 50, the detection unit 51, the derivation unit 52, the determination unit 53, the generation unit 54, and the display control unit 55, as illustrated in Fig. 4.

The image capturing device 19 captures specimen images 15 of liver specimens LVS of a subject S. The specimen images 15 are output from the image capturing device 19 to the drug discovery support device 10. In the drug discovery support device 10, the specimen images 15 received from the image capturing device 19 are stored in the storage 30 by the RW control unit 50. At this time, as illustrated in Fig. 5, the RW control unit 50 performs a resolution conversion process on the original specimen images 15 received from the image capturing device 19, that is, original images 15O, thereby generating analysis images 15A. Subsequently, the original images 15O and the analysis images 15A are associated with each other and stored in the storage 30.

In response to an instruction to display the specimen images 15 being provided by the user U via the input device 12, the RW control unit 50 reads and obtains the specimen images 15 designated by the display instruction from the storage 30 (step ST100). The specimen images 15 are output from the RW control unit 50 to the display control unit 55. As illustrated in Fig. 27, the specimen images 15 are displayed on the display 11 via the target designation screen 125 under the control of the display control unit 55 (step ST105).

In response to the selection frame 128 being set to a desired specimen image 15 on the target designation screen 125 and the analyze button 129 being selected by the user U (YES in step ST110), the RW control unit 50 reads and obtains, as a target specimen image 15T, the analysis image 15A of the specimen image 15 to which the selection frame 128 is set at that time from the storage 30 (step ST115). The target specimen image 15T is output from the RW control unit 50 to the detection unit 51, the generation unit 54, and the display control unit 55.

The RW control unit 50 reads the feature quantity extractor 41 and the detection reference information 42 from the storage 30, and outputs the read feature quantity extractor 41 and detection reference information 42 to the detection unit 51.

As illustrated in Fig. 6, the detection unit 51 divides the target specimen image 15T into multiple patch images 70. Subsequently, as illustrated in Fig. 7, the detection unit 51 extracts a feature quantity 72 from each patch image 70 by using the feature quantity extractor 41.

As illustrated in Fig. 13, the detection unit 51 calculates the distance D between the representative position of the reference feature quantities 72R and the position of the feature quantity 72. Subsequently, as illustrated in Fig. 14 and Fig. 15, the detection unit 51 compares the distance D with the detection threshold value 84 to detect whether a morphological abnormality has occurred in the liver specimen LVS in the patch image 70 (step ST120). The detection result 60 indicating whether a morphological abnormality has occurred in the liver specimen LVS in the patch image 70 is output from the detection unit 51 to the derivation unit 52.

The process of extracting the feature quantity 72 from the patch image 70, the process of calculating the distance D between the representative position of the reference feature quantities 72R and the position of the feature quantity 72, and the process of detecting whether a morphological abnormality has occurred in the liver specimen LVS in the patch image 70 are performed on all the patch images 70 of the target specimen image 15T. After the above-described processes have been performed on all the patch images 70, the process proceeds to step ST125.

As illustrated in Fig. 16, the derivation unit 52 derives the determination reference information 61 from the detection results 60 (step ST125). The determination reference information 61 is output from the derivation unit 52 to the determination unit 53 and the display control unit 55.

As illustrated in Fig. 17 and Fig. 18, the determination unit 53 determines, based on the determination threshold value 43 and the determination reference information 61, whether estimated morphological abnormality portions have been overdetected and whether estimated morphological abnormality portions have been underdetected (step ST130). The determination result 62 is output from the determination unit 53 to the generation unit 54.

In response to the determination unit 53 determining that estimated morphological abnormality portions have been overdetected or underdetected (YES in step ST135), the generation unit 54 generates the first cause identification reference information 631 or the second cause identification reference information 632 (step ST140) as illustrated in Fig. 20 to Fig. 26. To be specific, as illustrated in Fig. 20 to Fig. 22, the type of artifacts that may have been erroneously detected as an estimated morphological abnormality portion and the number of the identified artifacts are generated as the first cause identification reference information 631 or the second cause identification reference information 632. In addition, as illustrated in Fig. 23 and Fig. 24, the target histogram 95, the reference histogram 96, the Bhattacharyya distances, the hematoxylin component 102T and the eosin component 103T of the target specimen image 15T, and the hematoxylin component 102RR and the eosin component 103RR of the representative reference specimen image 15RR are generated as the first cause identification reference information 631 or the second cause identification reference information 632. Furthermore, as illustrated in Fig. 25 and Fig. 26, the cluster images 115 to 117 are generated as the first cause identification reference information 631. The first cause identification reference information 631 or the second cause identification reference information 632 is output from the generation unit 54 to the display control unit 55.

Under the control of the display control unit 55, the analysis result display screen 135 illustrated in Fig. 28 is displayed on the display 11 (step ST145). In response to the information display button 138 being selected by the user U (YES in step ST150), the information display screen 145 illustrated in Fig. 29 to Fig. 31 is displayed on the display 11 under the control of the display control unit 55 (step ST155). Accordingly, the first cause identification reference information 631 or the second cause identification reference information 632 is presented to the user U. In addition, a suppression process and/or an exclusion process is proposed to the user U by a message 155.

As described above, the CPU 32 of the drug discovery support device 10 includes the RW control unit 50, the detection unit 51, the derivation unit 52, the determination unit 53, and the display control unit 55. The RW control unit 50 obtains the target specimen image 15T of the liver specimen LVS of the subject S subjected to an evaluation test of the candidate substance 27 by reading the target specimen image 15T from the storage 30. The detection unit 51 detects, from among the patch images 70 obtained by dividing the target specimen image 15T, one or more patch images 70 where a morphological abnormality is estimated to have occurred (one or more estimated morphological abnormality portions). The derivation unit 52 derives the determination reference information 61 from the detection result 60 of each of the one or more estimated morphological abnormality portions. The determination unit 53 determines, based on the determination reference information 61, whether estimated morphological abnormality portions have been overdetected and whether estimated morphological abnormality portions have been underdetected. In response to a determination being made that estimated morphological abnormality portions have been overdetected, the display control unit 55 presents to the user U the first cause identification reference information 631 contributing to identifying the cause of the estimated morphological abnormality portions having been overdetected. On the other hand, in response to a determination being made that estimated morphological abnormality portions have been underdetected, the display control unit 55 presents to the user U the second cause identification reference information 632 contributing to identifying the cause of the estimated morphological abnormality portions having been underdetected.

The user U is able to browse the first cause identification reference information 631 or the second cause identification reference information 632 to identify the cause of the estimated morphological abnormality portions having been overdetected or underdetected, and perform an improvement process in accordance with the message 155. This makes it possible to contribute to improving the accuracy of detecting a morphological abnormality occurred in the liver specimen LVS in the specimen image 15.

As illustrated in Fig. 4 and so forth, the RW control unit 50 obtains a single target specimen image 15T by reading the target specimen image 15T from the storage 30. The detection unit 51 detects one or more estimated morphological abnormality portions from the single target specimen image 15T. The derivation unit 52 derives, as the determination reference information 61, a number ratio which is a numerical value representing the spatial disposition state of the estimated morphological abnormality portions in the single target specimen image 15T. Accordingly, the detection of estimated morphological abnormality portions and the derivation of a numerical value can be simply performed.

As illustrated in Fig. 17 and Fig. 18, the determination unit 53 compares the number ratio with the determination threshold value 43 set in advance, thereby determining whether estimated morphological abnormality portions have been overdetected and whether estimated morphological abnormality portions have been underdetected. Accordingly, the determination can be simply and accurately performed. In addition, the determination criterion is clear, and there is no possibility of erroneous determination.

As illustrated in Fig. 16, the numerical value is a numerical value related to the number of estimated morphological abnormality portions, more specifically, a number ratio. Accordingly, the derivation of the numerical value can be simply performed.

As illustrated in Fig. 20 to Fig. 22, the generation unit 54 identifies the type of artifact that may have been erroneously detected as an estimated morphological abnormality portion. As illustrated in Fig. 29, the display control unit 55 presents to the user U information regarding the type as the first cause identification reference information 631 and the second cause identification reference information 632. Accordingly, the user U is able to determine whether the cause of estimated morphological abnormality portions having been overdetected or underdetected is an artifact.

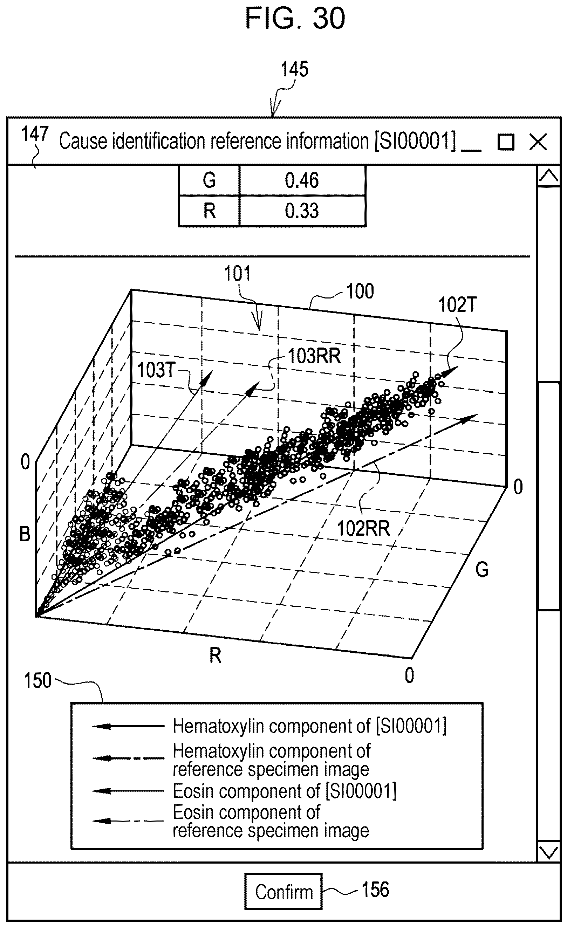

As illustrated in Fig. 29 and Fig. 30, the display control unit 55 presents to the user U the target histogram 95, the reference histogram 96, the table 97 of Bhattacharyya distances, the hematoxylin component 102T and the eosin component 103T of the target specimen image 15T, and the hematoxylin component 102RR and the eosin component 103RR of the representative reference specimen image 15RR as the first cause identification reference information 631 and the second cause identification reference information 632. Accordingly, the user U is able to determine whether the cause of estimated morphological abnormality portions having been overdetected or underdetected is the tint of the target specimen image 15T.

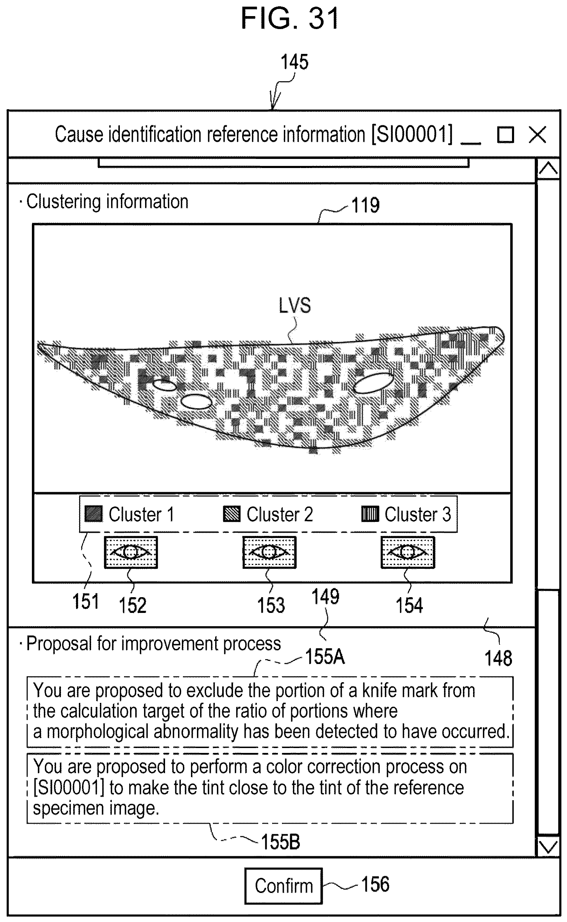

As illustrated in Fig. 25 and Fig. 26, in response to a determination being made that estimated morphological abnormality portions have been overdetected, the generation unit 54 performs a clustering process of defining a cluster to which each of the multiple estimated morphological abnormality portions detected from the single target specimen image 15T belongs. As illustrated in Fig. 31, the display control unit 55 presents to the user U the multiple cluster images 115 to 117 that are generated by processing the target specimen image 15T and that reflect the result of the clustering process as the first cause identification reference information 631. The cluster images 115 to 117 are images that enable multiple clusters to be identified by the display format 118 set in advance for each of the multiple clusters. Accordingly, the user U is able to determine whether the determination that estimated morphological abnormality portions have been overdetected is appropriate. It is rare that there is only one type of morphological abnormality, and there may be multiple types of morphological abnormalities. Thus, when the number of clusters is relatively small, for example, when the number of clusters is one, it can be determined that the determination that estimated morphological abnormality portions have been overdetected is appropriate. On the other hand, when the number of clusters is relatively large, it can be determined that the determination that estimated morphological abnormality portions have been overdetected is inappropriate.

The display control unit 55 proposes to the user U a suppression process of suppressing overdetection or underdetection of estimated morphological abnormality portions, by using the message 155. To be specific, the suppression process includes a process of changing the detection threshold value 84 of an estimated morphological abnormality portion, a process of correcting the color of the target specimen image 15T, and a process of changing the resolution of the target specimen image 15T. Accordingly, the user U performs an appropriate suppression process in accordance with the message 155, and thus the accuracy of detecting a morphological abnormality can be improved. The suppression process does not have to include all of the process of changing the detection threshold value 84, the process of correcting the color of the target specimen image 15T, and the process of changing the resolution of the target specimen image 15T, and may include at least one of these processes.

Here, it is assumed that the user U changes the resolution of the target specimen image 15T in accordance with the message 155E prompting the user U to change the resolution of the target specimen image 15T, to be specific, the user U uses the original image 15O instead of the analysis image 15A as the target specimen image 15T. In this case, the feature quantity extractor 41 is switched to the one for the original image 15O. That is, two types of feature quantity extractors 41 for the analysis image 15A and the original image 15O are prepared.

In addition, the display control unit 55 proposes to the user U an exclusion process of excluding, from the target of an evaluation test, an artifact portion that may have been erroneously detected as an estimated morphological abnormality portion, by using the message 155. Accordingly, the user U performs an appropriate exclusion process in accordance with the message 155, and thus the reliability of the evaluation test can be increased.

As illustrated in Fig. 6, Fig. 7, and Fig. 13, the detection unit 51 handles each of the multiple patch images 70 obtained by dividing the target specimen image 15T as a portion. The detection unit 51 compares the feature quantities 72 obtained by inputting the patch images 70 to the feature quantity extractor 41 with the reference feature quantities 72R obtained by inputting the reference patch images 70R of the liver specimen LVS regarded to be normal to the feature quantity extractor 41, and thus detects the one or more estimated morphological abnormality portions. Accordingly, an estimated morphological abnormality portion can be detected easily and accurately.

### Second Embodiment

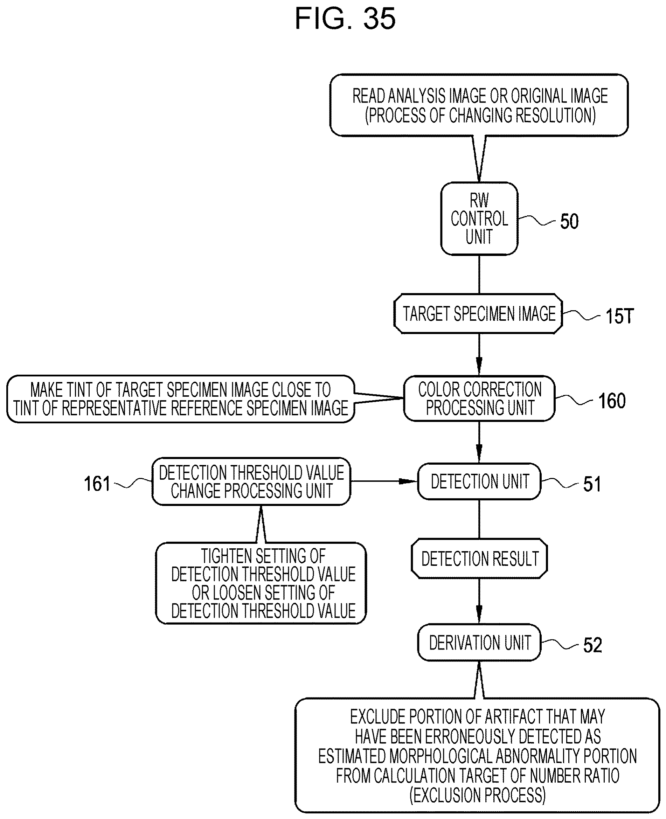

In the above-described first embodiment, a suppression process and an exclusion process are merely proposed to the user U by using the message 155, but the present disclosure is not limited thereto. As illustrated in Fig. 35 as an example, the CPU 32 may perform a suppression process and an exclusion process.

Referring to Fig. 35, a CPU of a drug discovery support device according to a second embodiment functions as a color correction processing unit 160 and a detection threshold value change processing unit 161 in addition to the processing units 50 to 55 according to the above-described first embodiment (the determination unit 53, the generation unit 54, and the display control unit 55 are not illustrated in Fig. 35). The color correction processing unit 160 is provided between the RW control unit 50 and the detection unit 51. The detection threshold value change processing unit 161 is connected to the detection unit 51.

When the tint of the target specimen image 15T and the tint of the representative reference specimen image 15RR are different from each other by a tint threshold value set in advance or more, the color correction processing unit 160 performs a color correction process on the target specimen image 15T. The color correction process is a process of making the tint of the target specimen image 15T close to the tint of the representative reference specimen image 15RR. To be specific, the color correction process is a process of making the Bhattacharyya distance between the target histogram 95 and the reference histogram 96 close to 0, and/or a process of making the hematoxylin component 102T and the eosin component 103T of the target specimen image 15T close to the hematoxylin component 102RR and the eosin component 103RR of the representative reference specimen image 15RR. The color correction processing unit 160 outputs the target specimen image 15T subjected to the color correction process to the detection unit 51, the generation unit 54, and the display control unit 55.

The detection threshold value change processing unit 161 performs a process of changing the detection threshold value 84 when the determination unit 53 determines that estimated morphological abnormality portions have been overdetected or underdetected, there is no type of artifact identified as having a possibility of having been erroneously detected as an estimated morphological abnormality portion, and the tints of the target specimen image 15T and the representative reference specimen image 15RR are not different from each other by the tint threshold value or more. To be specific, when a determination is made that estimated morphological abnormality portions have been overdetected, the detection threshold value change processing unit 161 resets the detection threshold value 84 to a larger value. On the other hand, when a determination is made that estimated morphological abnormality portions have been underdetected, the detection threshold value change processing unit 161 resets the detection threshold value 84 to a smaller value. The degree of increasing or decreasing the detection threshold value 84 may be uniform or may be changed in accordance with, for example, the difference between the number ratio and the determination threshold value 43. In the case of changing the degree in accordance with the difference between the number ratio and the determination threshold value 43, the degree is increased when the difference between the number ratio and the determination threshold value 43 is relatively large, and the degree is decreased when the difference is relatively small.

The RW control unit 50 reads the original image 15O, instead of the analysis image 15A, as the target specimen image 15T from the storage 30 when the determination unit 53 determines that estimated morphological abnormality portions have been underdetected, there is no type of artifact identified as having a possibility of having been erroneously detected as an estimated morphological abnormality portion, and the tints of the target specimen image 15T and the representative reference specimen image 15RR are not different from each other by the tint threshold value or more. That is, the RW control unit 50 performs a process of changing the resolution of the target specimen image 15T.

When there is a type of artifact identified as having a possibility of having been erroneously detected as an estimated morphological abnormality portion, the derivation unit 52 excludes the patch image 70 including the artifact identified as having a possibility of having been erroneously detected as an estimated morphological abnormality portion from the calculation target of the number ratio. That is, the derivation unit 52 performs an exclusion process of excluding the portion of the artifact that may have been erroneously detected as an estimated morphological abnormality portion from the target of the evaluation test.

As described above, in the second embodiment, the RW control unit 50, the color correction processing unit 160, and the detection threshold value change processing unit 161 perform a suppression process of suppressing overdetection or underdetection of estimated morphological abnormality portions. To be specific, the suppression process includes a process of changing the detection threshold value 84 of an estimated morphological abnormality portion by the detection threshold value change processing unit 161, a process of correcting the color of the target specimen image 15T by the color correction processing unit 160, and a process of changing the resolution of the target specimen image 15T by the RW control unit 50. Accordingly, the accuracy of detecting a morphological abnormality can be improved without troubling the user U.

In addition, the derivation unit 52 performs an exclusion process of excluding the portion of an artifact that may have been erroneously detected as an estimated morphological abnormality portion from a target of an evaluation test. Accordingly, the reliability of the evaluation test can be increased without troubling the user U.

As in the above-described first embodiment, a suppression process and/or an exclusion process may be performed after proposing a suppression process and/or an exclusion process to the user U. In this case, a suppression process and/or an exclusion process may be performed only when an instruction has been provided from the user U, or a suppression process and/or an exclusion process may be performed without waiting for an instruction from the user U.

### Third Embodiment

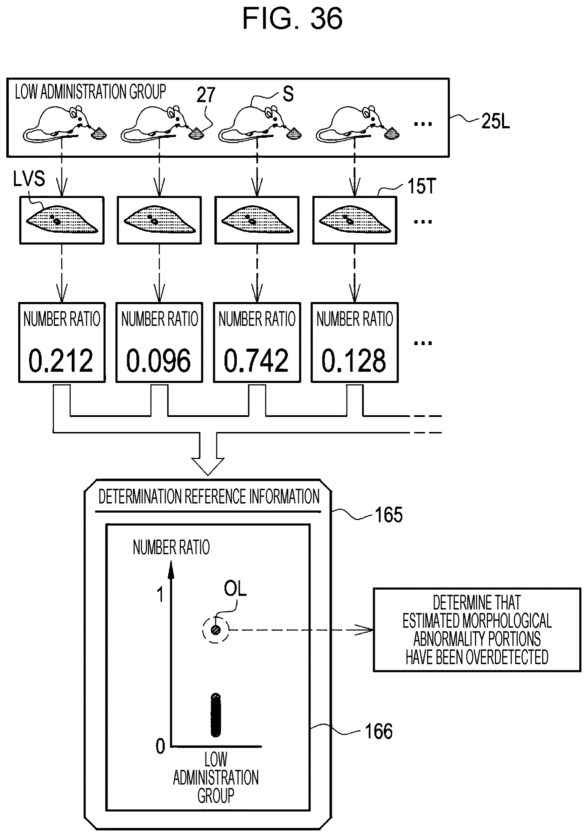

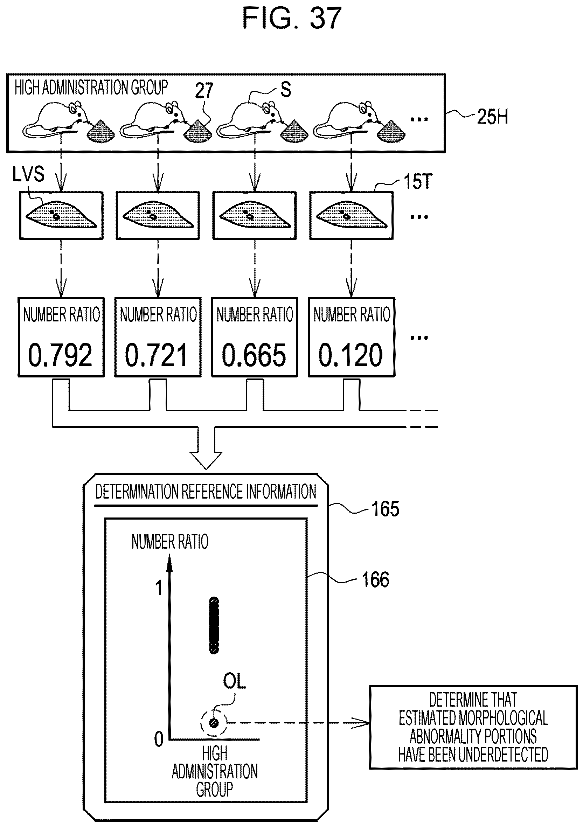

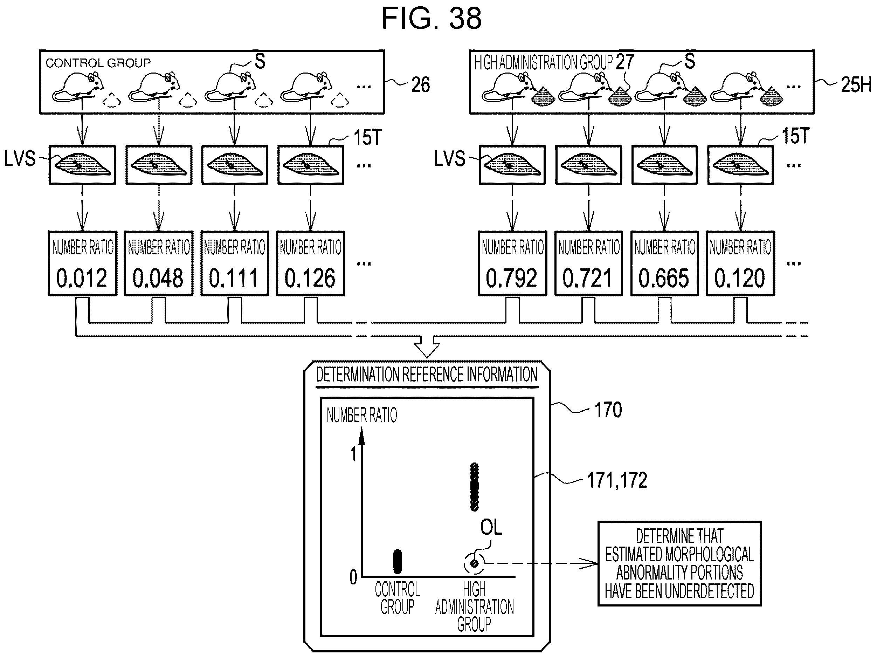

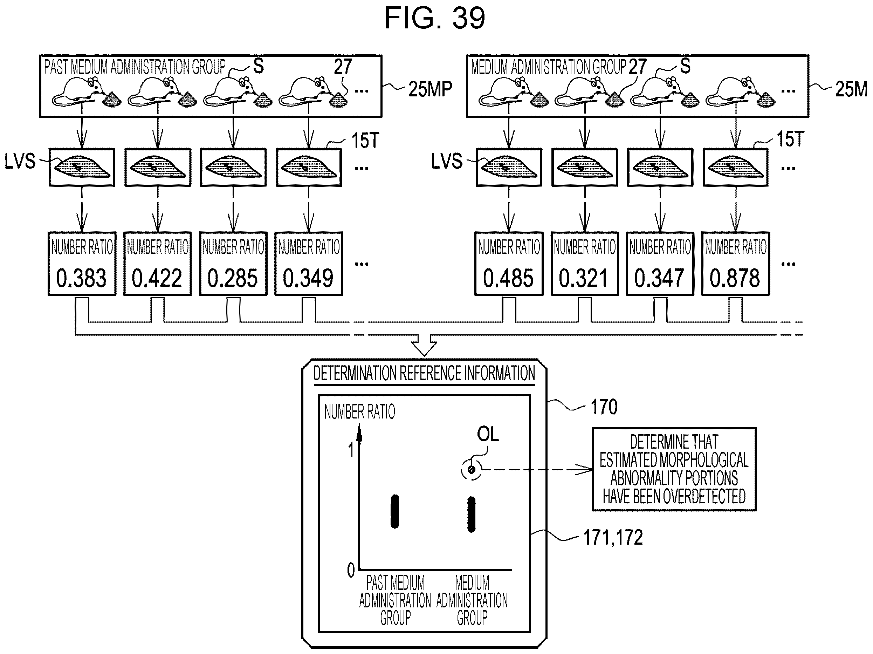

In the above-described first embodiment, a process is performed using a single target specimen image 15T. In a third embodiment illustrated in Fig. 36 to Fig. 39 as an example, a process is performed using multiple target specimen images 15T. Fig. 36 and Fig. 37 each illustrate an example in which a process is performed using target specimen images 15T of a liver specimen LVS of multiple subjects S belonging to the same group. On the other hand, Fig. 38 and Fig. 39 each illustrate an example in which a process is performed using target specimen images 15T of a liver specimen LVS of multiple subjects S belonging to different groups.

In the case of Fig. 36, the RW control unit 50 obtains the target specimen images 15T of the liver specimen LVS of the multiple subjects S belonging to the low administration group 25L by reading the target specimen images 15T from the storage 30. The detection unit 51 detects one or more estimated morphological abnormality portions from each of the multiple target specimen images 15T. The derivation unit 52 derives a number ratio of each of the multiple target specimen images 15T. The derivation unit 52 derives a distribution 166 of the number ratios as determination reference information 165.

The determination unit 53 detects an outlier OL from among the number ratios constituting the distribution 166. The outlier OL can be detected using a method using an interquartile range, a Smirnov-Grubbs test, a clustering process, or the like. In the distribution 166 of the number ratios in this case, because the population is the low administration group 25L, it is considered that the majority gather in a region where the value is relatively small, and that the outlier OL appears in a region where the value is relatively large. Thus, the determination unit 53 determines that estimated morphological abnormality portions have been overdetected in the target specimen image 15T where the number ratio is the outlier OL.

Fig. 37 illustrates an example of using the target specimen images 15T of the liver specimen LVS of the multiple subjects S belonging to the high administration group 25H. In the distribution 166 of the number ratios in this case, because the population is the high administration group 25H, it is considered that the majority gather in a region where the value is relatively large, and that the outlier OL appears in a region where the value is relatively small, contrary to the case of Fig. 36. Thus, the determination unit 53 determines that estimated morphological abnormality portions have been underdetected in the target specimen image 15T where the number ratio is the outlier OL.

In the case of Fig. 38, the RW control unit 50 obtains the target specimen images 15T of the liver specimen LVS of the multiple subjects S belonging to the control group 26 and the target specimen images 15T of the liver specimen LVS of the multiple subjects S belonging to the high administration group 25H by reading the target specimen images 15T from the storage 30. The detection unit 51 detects one or more estimated morphological abnormality portions from each of the multiple target specimen images 15T. The derivation unit 52 derives a number ratio of each of the multiple target specimen images 15T. The derivation unit 52 derives a distribution 171 of the number ratios of the control group 26 and a distribution 172 of the number ratios of the high administration group 25H as determination reference information 170.

The determination unit 53 detects an outlier OL from among the number ratios constituting the distribution 172 of the high administration group 25H. In addition, the determination unit 53 refers to the distribution 171 of the control group 26 to determine the validity of the outlier OL detected from the distribution 172 of the high administration group 25H. To be specific, the determination unit 53 calculates the difference between a typical value, such as an average value or a median value, of the number ratios of the control group 26 and the outlier OL detected from the distribution 172 of the high administration group 25H. When the difference is smaller than a threshold value set in advance, the determination unit 53 determines that the outlier OL is valid. In this case, the determination unit 53 determines that estimated morphological abnormality portions have been underdetected in the target specimen image 15T where the number ratio is the outlier OL.

Fig. 39 illustrates an example of using the target specimen images 15T of the liver specimen LVS of the multiple subjects S belonging to a past medium administration group 25MP and the target specimen images 15T of the liver specimen LVS of the multiple subjects S belonging to the medium administration group 25M. Here, the past medium administration group 25MP is an administration group to which a candidate substance identical or similar to the candidate substance 27 of the current evaluation test was administered in the same dose as in the medium administration group 25M in a past evaluation test. The similar candidate substance is a candidate substance similar in composition to the candidate substance 27. The derivation unit 52 derives the distribution 171 of the number ratios of the past medium administration group 25MP and the distribution 172 of the number ratios of the medium administration group 25M as the determination reference information 170. In the distributions 171 and 172 of the number ratios in this case, because the population is the past medium administration group 25MP and the medium administration group 25M, it is considered that the majority gather in a region where the value is medium, and that the outlier OL appears in either a region where the value is relatively small or a region where the value is relatively large.

In this case, the determination unit 53 detects the outlier OL from among the number ratios constituting the distribution 172 of the medium administration group 25M. In addition, the determination unit 53 refers to the distribution 171 of the past medium administration group 25MP to determine the validity of the outlier OL detected from the distribution 172 of the medium administration group 25M. To be specific, the determination unit 53 calculates the difference between a typical value, such as an average value or a median value, of the number ratios of the past medium administration group 25MP and the outlier OL detected from the distribution 172 of the medium administration group 25M. When the difference is larger than or equal to a threshold value set in advance, the determination unit 53 determines that the outlier OL is valid.

When the outlier OL is in a region where the value is relatively small and the outlier OL is valid, the determination unit 53 determines that estimated morphological abnormality portions have been underdetected in the target specimen image 15T where the number ratio is the outlier OL. On the other hand, as illustrated in the figure, when the outlier OL is in a region where the value is relatively large and the outlier OL is valid, the determination unit 53 determines that estimated morphological abnormality portions have been overdetected in the target specimen image 15T where the number ratio is the outlier OL. Although Fig. 36 to Fig. 39 illustrate a case where there is only one outlier OL, the number of outliers OL is not limited to one. Multiple outliers OL may be detected.

As described above, in the third embodiment, the RW control unit 50 obtains multiple target specimen images 15T by reading the target specimen images 15T from the storage 30. The detection unit 51 detects one or more estimated morphological abnormality portions from each of the multiple target specimen images 15T. The derivation unit 52 derives, as the determination reference information 165 or 170, the distribution 166, 171, or 172 of the number ratios which are numerical values each representing the spatial disposition state of the estimated morphological abnormality portions in a corresponding one of the multiple target specimen images 15T. Accordingly, it is possible to determine whether estimated morphological abnormality portions have been overdetected and whether estimated morphological abnormality portions have been underdetected, by comparison using the multiple target specimen images 15T instead of a single target specimen image 15T. The time taken to make a determination on the multiple target specimen images 15T can be significantly reduced as compared with the case of determining, for each target specimen image 15T, whether estimated morphological abnormality portions have been overdetected and whether estimated morphological abnormality portions have been underdetected.

As illustrated in Fig. 36 and Fig. 37, the multiple target specimen images 15T are images of the liver specimen LVS of the multiple subjects S belonging to the same group. The determination unit 53 detects the outlier OL from among the number ratios constituting the distribution 166, and thus determines whether estimated morphological abnormality portions have been overdetected and whether estimated morphological abnormality portions have been underdetected. Accordingly, it is possible to determine in a short time, for the multiple target specimen images 15T of the liver specimen LVS of the multiple subjects S belonging to the same group, whether estimated morphological abnormality portions have been overdetected and whether estimated morphological abnormality portions have been underdetected.

As illustrated in Fig. 38 and Fig. 39, the multiple target specimen images 15T are images of the liver specimen LVS of the multiple subjects S belonging to different groups. The derivation unit 52 derives the distributions 171 and 172 for the respective different groups. The determination unit 53 detects the outlier OL from among the number ratios constituting the distribution 172 of the multiple distributions 171 and 172 derived for the respective different groups, refers to the distribution 171 other than the distribution 172, and thus determines whether estimated morphological abnormality portions have been overdetected and whether estimated morphological abnormality portions have been underdetected. Accordingly, it is possible to detect the outlier OL having validity, and enhance the validity of the determination of whether estimated morphological abnormality portions have been overdetected and whether estimated morphological abnormality portions have been underdetected.

The different groups are the administration group 25 to which the candidate substance 27 is administered and the control group 26 to which the candidate substance 27 is not administered, such as the high administration group 25H and the control group 26 illustrated in Fig. 38. With this combination, the validity of the outlier OL detected from the distribution 172 of the number ratios of the administration group 25 can be determined with reference to the distribution 171 of the number ratios of the control group 26. The different groups are the administration group 25 to which the candidate substance 27 is administered and a past administration group to which a candidate substance identical or similar to the candidate substance 27 was administered in a past evaluation test, such as the medium administration group 25M and the past medium administration group 25MP illustrated in Fig. 39. With this combination, the validity of the outlier OL detected from the distribution 172 of the number ratios of the administration group 25 can be determined with reference to the distribution 171 of the number ratios of the past administration group.

The different groups may be, for example, the low administration group 25L and the medium administration group 25M, or the medium administration group 25M and the high administration group 25H. Alternatively, the different groups may be, for example, the high administration group 25H and a past high administration group. Furthermore, the different groups may be three or more groups such as the low administration group 25L, the high administration group 25H, and the control group 26.

### Fourth Embodiment

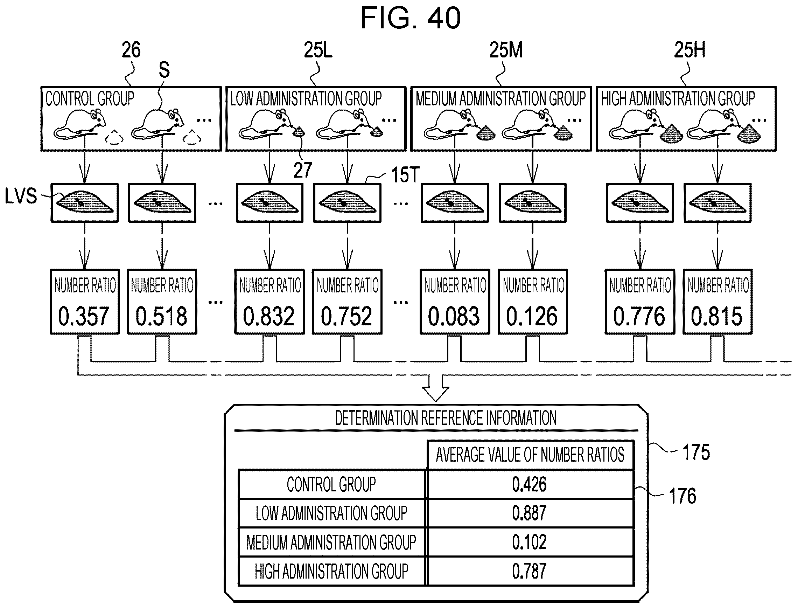

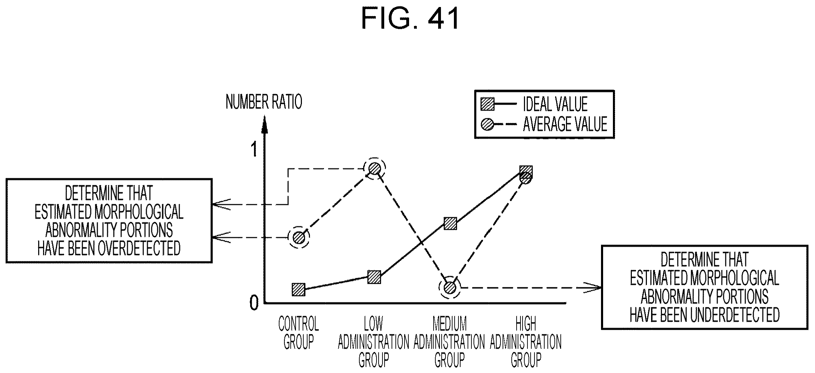

In a fourth embodiment illustrated in Fig. 40 and Fig. 41 as an example, a representative value of number ratios is derived for each of different groups. The representative value of each of the different groups is compared with an ideal value of the number ratios that is set in advance for a corresponding one of the different groups, and accordingly it is determined whether estimated morphological abnormality portions have been overdetected and whether estimated morphological abnormality portions have been underdetected.

As illustrated in Fig. 40, the RW control unit 50 obtains the multiple target specimen images 15T of the liver specimen LVS of the multiple subjects S belonging to the low administration group 25L, the medium administration group 25M, the high administration group 25H, and the control group 26 by reading the target specimen images 15T from the storage 30. The detection unit 51 detects one or more estimated morphological abnormality portions from each of the multiple target specimen images 15T. The derivation unit 52 derives the number ratio of each of the multiple target specimen images 15T. The derivation unit 52 derives, as determination reference information 175, the average value of the number ratios for each of the low administration group 25L, the medium administration group 25M, the high administration group 25H, and the control group 26 as illustrated in a table 176. The average value of the number ratios is an example of "a representative value of numerical values" according to the technology of the present disclosure. The representative value may be a median value, a mode value, or the like instead of an average value.

As illustrated in Fig. 41 as an example, the determination unit 53 compares the average value of the number ratios of each group with the ideal value of the number ratios set in advance for each group. When the average value is larger than the ideal value and the difference between the average value and the ideal value is larger than or equal to a determination threshold value set in advance, the determination unit 53 determines that estimated morphological abnormality portions have been overdetected in the target specimen images 15T of the group. When the average value is smaller than the ideal value and the difference between the average value and the ideal value is larger than or equal to the determination threshold value, the determination unit 53 determines that estimated morphological abnormality portions have been underdetected in the target specimen images 15T of the group. Fig. 41 illustrates a case where a determination is made that estimated morphological abnormality portions have been overdetected in the target specimen images 15T of the low administration group 25L and the control group 26. Fig. 41 also illustrates a case where a determination is made that estimated morphological abnormality portions have been underdetected in the target specimen images 15T of the medium administration group 25M.

In this case, the display control unit 55 switchably displays, on the information display screen 145, multiple pieces of cause identification reference information 63 for the multiple target specimen images 15T of the group for which a determination is made that estimated morphological abnormality portions have been overdetected or underdetected.

As described above, in the fourth embodiment, the RW control unit 50 obtains the multiple target specimen images 15T of the liver specimen LVS of the multiple subjects S belonging to different groups by reading the target specimen images 15T from the storage 30. The detection unit 51 detects one or more estimated morphological abnormality portions from each of the multiple target specimen images 15T. The derivation unit 52 derives, as the determination reference information 175, for each of the different groups, the average value of number ratios which is a representative value of numerical values each representing the spatial disposition state of estimated morphological abnormality portions in a corresponding one of the multiple target specimen images 15T. Accordingly, it is possible to determine whether estimated morphological abnormality portions have been overdetected and whether estimated morphological abnormality portions have been underdetected, by comparison using the multiple target specimen images 15T instead of a single target specimen image 15T. The time taken to make a determination on the multiple target specimen images 15T can be significantly reduced as compared with the case of determining, for each target specimen image 15T, whether estimated morphological abnormality portions have been overdetected and whether estimated morphological abnormality portions have been underdetected.

As illustrated in Fig. 41, the determination unit 53 compares the average value of the number ratios of each of the different groups with the ideal value of the number ratios that is set in advance for a corresponding one of the different groups, and thus determines whether estimated morphological abnormality portions have been overdetected and whether estimated morphological abnormality portions have been underdetected. Accordingly, it is possible to determine in a short time, for the multiple target specimen images 15T of the liver specimen LVS of the multiple subjects S belonging to a single group, whether estimated morphological abnormality portions have been overdetected and whether estimated morphological abnormality portions have been underdetected.

The different groups are the administration group 25 to which the candidate substance 27 is administered and the control group 26 to which the candidate substance 27 is not administered. Accordingly, it is possible to determine in a short time, for the multiple target specimen images 15T of the liver specimen LVS of the multiple subjects S belonging to the administration group 25 and the control group 26, whether estimated morphological abnormality portions have been overdetected and whether estimated morphological abnormality portions have been underdetected.

The administration group 25 includes the low administration group 25L, the medium administration group 25M, and the high administration group 25H that are different from each other in a dose of the candidate substance 27. Accordingly, it is possible to determine in a short time, for the multiple target specimen images 15T of the liver specimen LVS of the multiple subjects S belonging to the low administration group 25L, the medium administration group 25M, and the high administration group 25H, whether estimated morphological abnormality portions have been overdetected and whether estimated morphological abnormality portions have been underdetected.

The different groups may be two groups of the administration group 25 and the control group 26. Alternatively, the different groups may be three groups of the low administration group 25L, the medium administration group 25M, and the high administration group 25H except for the control group 26.

The process of the fourth embodiment may be performed to estimate a group that is likely to include the target specimen image 15T where estimated morphological abnormality portions have been overdetected or underdetected. Subsequently, the process of the above-described first embodiment or second embodiment may be performed on the target specimen images 15T of the estimated group to identify the target specimen image 15T where estimated morphological abnormality portions have been overdetected or underdetected.

### Modification 1

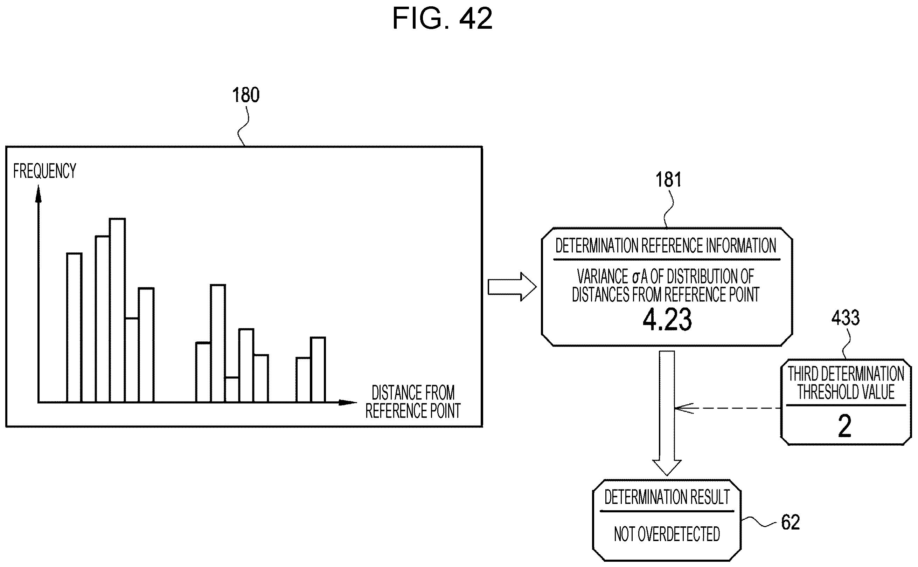

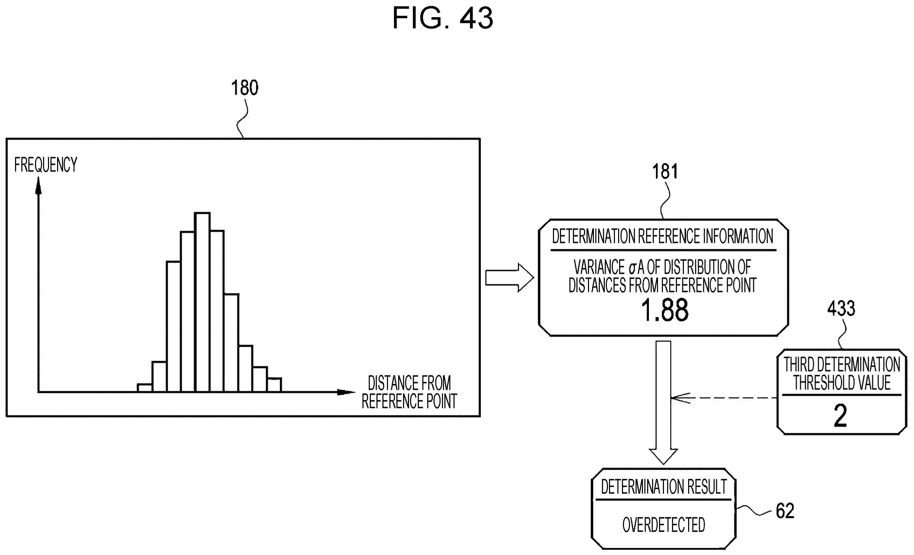

The numerical value representing the spatial disposition state of estimated morphological abnormality portions in the case of using a single target specimen image 15T is not limited to a numerical value related to the number of estimated morphological abnormality portions such as the number ratio exemplified in each of the above-described embodiments. As illustrated in Fig. 42 and Fig. 43 as an example, the derivation unit 52 generates a distribution 180 of the distances from a reference point to detected estimated morphological abnormality portions, and derives a variance σA of the distribution 180 as a numerical value representing the spatial disposition state of the estimated morphological abnormality portions. The derived variance σA may be used as determination reference information 181 together with the number ratio. The reference point is, for example, the center point of the target specimen image 15T.

In many cases, artifacts that may be erroneously detected as an estimated morphological abnormality portion concentrate in a specific region. Thus, in the case of the target specimen image 15T where artifacts have occurred, the variance σA of the distribution 180 tends to be small. Accordingly, when the number ratio is larger than or equal to the first determination threshold value 431, the determination unit 53 further performs a determination using the variance σA. To be specific, the determination unit 53 compares the variance σA with a third determination threshold value 433 set in advance. When the variance σA is larger than or equal to the third determination threshold value 433 as illustrated in Fig. 42, the determination unit 53 determines that estimated morphological abnormality portions have not been overdetected, and outputs the determination result 62 indicating the fact. On the other hand, when the variance σA is smaller than the third determination threshold value 433 as illustrated in Fig. 43, the determination unit 53 determines that estimated morphological abnormality portions have been overdetected, and outputs the determination result 62 indicating the fact. Instead of the variance σA, the standard deviation of the distribution 180 may be derived as a numerical value representing the spatial disposition state of estimated morphological abnormality portions.

### Modification 2

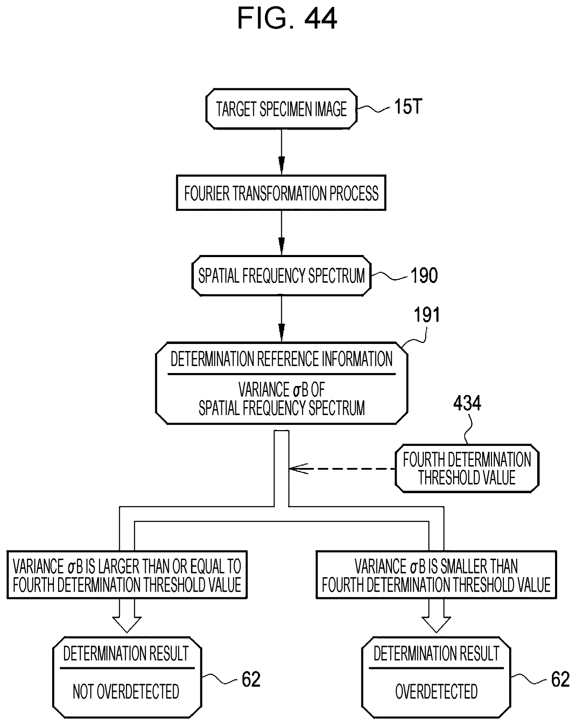

As illustrated in Fig. 44 as an example, the derivation unit 52 performs a Fourier transformation process on the target specimen image 15T to derive a spatial frequency spectrum 190 of the target specimen image 15T and derive a variance σB of the spatial frequency spectrum 190 as a numerical value representing the spatial disposition state of estimated morphological abnormality portions. The derived variance σB may be used as determination reference information 191 together with the number ratio.

The image capturing device 19 captures specimen images 15 of multiple regions obtained through division. Thus, in some of the specimen images 15, periodic boundaries between multiple regions are conspicuous. Such a boundary is a kind of artifact that may be erroneously detected as an estimated morphological abnormality portion. Thus, in the case of the target specimen image 15T where an artifact such as the forgoing boundary has occurred, the variance σB of the spatial frequency spectrum 190 tends to be small. Accordingly, when the number ratio is larger than or equal to the first determination threshold value 431, the determination unit 53 further performs a determination using the variance σB. To be specific, the determination unit 53 compares the variance σB with a fourth determination threshold value 434 set in advance. When the variance σB is larger than or equal to the fourth determination threshold value 434 as indicated by a left arrow, the determination unit 53 determines that estimated morphological abnormality portions have not been overdetected, and outputs the determination result 62 indicating the fact. On the other hand, when the variance σB is smaller than the fourth determination threshold value 434 as indicated by a right arrow, the determination unit 53 determines that estimated morphological abnormality portions have been overdetected, and outputs the determination result 62 indicating the fact. Instead of the variance σB, the standard deviation of the spatial frequency spectrum 190 may be derived as a numerical value representing the spatial disposition state of estimated morphological abnormality portions. As described above, as the numerical value representing the spatial disposition state of estimated morphological abnormality portions, various numerical values can be used instead of a numerical value related to the number of estimated morphological abnormality portions such as a number ratio.

In addition to the reference patch image 70R of the liver specimen LVS regarded to be normal, the patch image 70 of the liver specimen LVS where a morphological abnormality has occurred may also be used as the learning reference patch image 70RL. The patch image 70 of the liver specimen LVS where a morphological abnormality has occurred is obtained from, for example, a past administration group constituted by multiple subjects S to which a candidate substance was administered in a past evaluation test. Accordingly, the autoencoder 75 and thus the feature quantity extractor 41 is capable of learning about the liver specimens LVS having features of more various shapes and textures. As a result, the feature quantity extractor 41 is capable of extracting a feature quantity 72 that better represents the features of the shape and texture of the liver specimen LVS.

The patch image 70 of the liver specimen LVS where a morphological abnormality has occurred is not limited to an image obtained from the subject S constituting the exemplified past administration group. A morphological abnormality may occur also in the subject S constituting the past control group 26P. Thus, it does not matter whether the subject S belongs to the past control group 26P or the past administration group as long as the patch image 70 has the liver specimen LVS where a morphological abnormality has occurred. Furthermore, the patch image 70 of the liver specimen LVS where a morphological abnormality has occurred may be an image obtained from the subject S to which various stresses are applied to intentionally cause a morphological abnormality. The patch image 70 of the liver specimen LVS where a morphological abnormality has occurred may be an image artificially created by processing the patch image 70 of a normal liver specimen LVS.

Instead of the encoder unit 76 of the autoencoder 75, an encoder unit of a convolutional neural network that outputs a class determination result in accordance with an input of the patch image 70 may be diverted to be used as the feature quantity extractor 41. The class determination result is, for example, a result of determining one type of morphological abnormality that has occurred in the liver specimen LVS in the patch image 70 from among multiple types such as hyperplasia, infiltration, stasis, and inflammation.

The machine learning model diverted to be used as the feature quantity extractor 41 is not limited to the exemplified autoencoder 75 and convolutional neural network. A generator of generative adversarial networks (GAN) may be diverted to be used as the feature quantity extractor 41. A machine learning model that does not have a convolutional layer, such as a vision transformer (ViT), may be diverted to be used as the feature quantity extractor 41.

Contrastive learning may be performed in which learning is performed such that the distance in a feature quantity space between feature quantities derived from the same image is short and the distance in the feature quantity space between feature quantities derived from different images is long. As the contrastive learning, for example, a learning method such as a simple framework for contrastive learning of visual representations (SimCLR) is known. Alternatively, a learning method of not using the foregoing pair of different images (also referred to as a negative sample), such as bootstrap your own latent (BYOL), may be used. The distribution of extracted feature quantities may be restricted to a distribution on a unit sphere or a distribution following a standard normal distribution.

### Fifth Embodiment

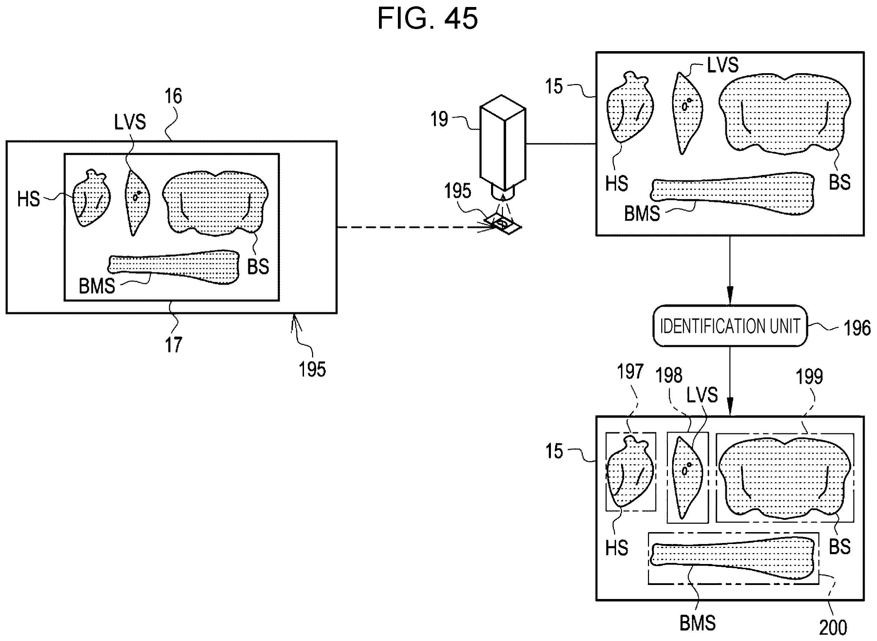

As illustrated in Fig. 45 as an example, in a fifth embodiment, a specimen slide 195 is handled in which tissue specimens of multiple types of organs are placed on a single glass slide 16. Fig. 45 illustrates a case where a heart specimen HS, a brain specimen BS, and a bone marrow specimen BMS are placed in addition to the liver specimen LVS. In this case, the specimen image 15 includes the heart specimen HS, the brain specimen BS, and the bone marrow specimen BMS in addition to the liver specimen LVS.

A CPU of a drug discovery support device according to the fifth embodiment functions as an identification unit 196 in addition to the processing units 50 to 55 according to the above-described first embodiment. The identification unit 196 identifies the tissue specimens of the respective organs from the specimen image 15 by using, for example, a template for identifying the tissue specimens of the respective organs or a machine learning model. The identification unit 196 outputs, as an identification result, coordinate information of frames 197 to 200 surrounding the tissue specimens of the respective organs. The frame 197 is a frame surrounding the heart specimen HS, and the frame 198 is a frame surrounding the liver specimen LVS. The frame 199 is a frame surrounding the brain specimen BS, and the frame 200 is a frame surrounding the bone marrow specimen BMS.

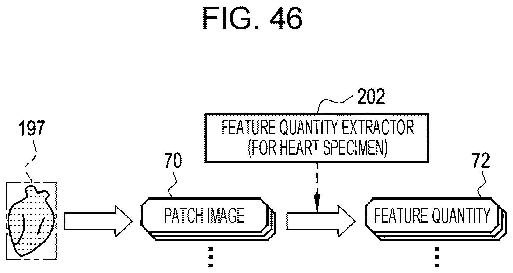

Fig. 46 illustrates a state in which the detection unit 51 divides the heart specimen HS in the frame 197 into multiple patch images 70, and extracts feature quantities 72 from the patch images 70 by using a feature quantity extractor 202 for the heart specimen HS. The detection unit 51 also extracts feature quantities 72 of the liver specimen LVS in the frame 198, the brain specimen BS in the frame 199, and the bone marrow specimen BMS in the frame 200 by using a dedicated feature quantity extractor. That is, the detection unit 51 extracts the feature quantities 72 of the tissue specimens of the respective organs identified by the identification unit 196. The subsequent process is the same as the process described in the first embodiment and so forth except that a determination or the like is performed on each of the tissue specimens of the organs in the target specimen image 15T, and thus the illustration and description thereof will be omitted.

As described above, in the fifth embodiment, the specimen image 15 is an image obtained by imaging the specimen slide 195 on which tissue specimens of multiple types of organs are placed. The identification unit 196 identifies the tissue specimens of the respective organs from the specimen image 15. The detection unit 51 extracts the feature quantities 72 of each of the identified tissue specimens of the respective organs. Thus, it is possible to cope with the specimen slide 195 on which tissue specimens of multiple types of organs are placed. The specimen slide 195 on which tissue specimens of multiple types of organs are placed as in the present embodiment is more typical than the specimen slide 18 on which a tissue specimen of a single organ is placed as in the above-described first embodiment. Thus, it is possible to perform a process in accordance with a more typical operation. The frames 197 to 200 indicating the tissue specimens of the respective organs in the specimen image 15 may be defined by an operation of the user U.

The feature quantity 72 is not limited to the one extracted by the feature quantity extractor 41. The feature quantity 72 may be an average value, a maximum value, a minimum value, a mode value, a variance, or the like of the pixel values of the patch image 70.

The organ is not limited to the exemplified liver LV. The organ may be the stomach, the lungs, the small intestine, the large intestine, or the like. The subject S is not limited to a rat. The subject S may be a mouse, a guinea pig, a gerbil, a hamster, a ferret, a rabbit, a dog, a cat, a monkey, or the like.

The drug discovery support device 10 may be a personal computer installed in a drug development facility as illustrated in Fig. 1, or may be a server computer installed in a data center independent of the drug development facility.

When the drug discovery support device 10 is constituted by a server computer, the specimen images 15 are transmitted from personal computers installed in individual drug development facilities to the server computer via a network such as the Internet. The server computer distributes various screens including the target designation screen 125 to the personal computers in a format of screen data for web delivery created in a markup language such as an Extensible Markup Language (XML). Each personal computer reproduces, based on the screen data, a screen to be displayed on a web browser, and displays the reproduced screen on a display. Instead of the XML, another data description language such as JavaScript (registered trademark) Object Notation (JSON) may be used.

The drug discovery support device 10 according to the technology of the present disclosure can be widely used throughout all stages of drug development, from the setting of a drug discovery target in the earliest stage to a clinical trial in the final stage.

The candidate substance 27 is not limited to the exemplified drug. The candidate substance 27 may be another chemical substance such as an agricultural chemical or a radioactive substance.

The hardware configuration of the computer constituting the drug discovery support device 10 according to the technology of the present disclosure can be modified in various ways. For example, the drug discovery support device 10 may be constituted by multiple computers separated as hardware for the purpose of improving the processing capability and reliability. For example, the functions of the detection unit 51 and the derivation unit 52 and the functions of the determination unit 53 and the generation unit 54 may be implemented by two computers in a distributed manner. In this case, the two computers constitute the drug discovery support device 10.

As described above, the hardware configuration of the computer of the drug discovery support device 10 can be changed as appropriate in accordance with a required performance such as processing capability, safety, and reliability. Furthermore, not only the hardware but also the application program such as the operation program 40 can be duplicated or stored in multiple storages in a distributed manner for the purpose of ensuring safety and reliability.

In each of the above-described embodiments, for example, the following various types of processors may be used as the hardware structures of the processing units that execute various processes, such as the RW control unit 50, the detection unit 51, the derivation unit 52, the determination unit 53, the generation unit 54, the display control unit 55, the color correction processing unit 160, the detection threshold value change processing unit 161, and the identification unit 196. The various types of processors include, as described above, the CPU 32, which is a general-purpose processor that executes software (the operation program 40) and functions as various processing units; a programmable logic device (PLD), which is a processor whose circuit configuration is changeable after manufacturing, such as a field programmable gate array (FPGA); a dedicated electric circuit, which is a processor having a circuit configuration designed specifically for performing specific processing, such as an application specific integrated circuit (ASIC); and the like.

A single processing unit may be constituted by one of these various types of processors or may be constituted by a combination of two or more processors of the same type or different types (for example, a combination of multiple FPGAs, and/or a combination of a CPU and an FPGA). Multiple processing units may be constituted by a single processor.

Examples of constituting multiple processing units by a single processor are as follows. First, as represented by a computer of a client or server, a single processor is constituted by a combination of one or more CPUs and software, and the processor functions as multiple processing units. Secondly, as represented by a system on chip (SoC), a processor in which a single integrated circuit (IC) chip implements the function of an entire system including multiple processing units is used. In this way, various types of processing units are constituted by using one or more of the above-described various types of processors as a hardware structure.

Furthermore, as the hardware structure of the various types of processors, more specifically, electric circuitry formed by combining circuit elements such as semiconductor elements may be used.

From the above description, the technology described in the following appendices can be grasped.

### Appendix 1

A drug discovery support device including a processor,
the processor being configured to:
obtain a specimen image of a tissue specimen of an organ of a subject subjected to an evaluation test of a candidate substance;
detect, from among portions of the specimen image, one or more estimated morphological abnormality portions where a morphological abnormality is estimated to have occurred;
derive determination reference information from a detection result of each of the one or more estimated morphological abnormality portions;
determine, based on the determination reference information, whether the one or more estimated morphological abnormality portions have been overdetected and whether the one or more estimated morphological abnormality portions have been underdetected;
in response to a determination being made that the one or more estimated morphological abnormality portions have been overdetected, present to a user first cause identification reference information contributing to identifying a cause of the one or more estimated morphological abnormality portions having been overdetected; and
in response to a determination being made that the one or more estimated morphological abnormality portions have been underdetected, present to the user second cause identification reference information contributing to identifying a cause of the one or more estimated morphological abnormality portions having been underdetected.

### Appendix 2

The drug discovery support device according to appendix 1, wherein the processor is configured to:
obtain the specimen image, the specimen image including a single specimen image;
detect the one or more estimated morphological abnormality portions from the single specimen image; and
derive, as the determination reference information, a numerical value representing a spatial disposition state of the one or more estimated morphological abnormality portions in the single specimen image.

### Appendix 3

The drug discovery support device according to appendix 2, wherein the processor is configured to:
compare the numerical value with a determination threshold value set in advance, and thus determine whether the one or more estimated morphological abnormality portions have been overdetected and whether the one or more estimated morphological abnormality portions have been underdetected.

### Appendix 4

The drug discovery support device according to appendix 2 or appendix 3, wherein the numerical value is a numerical value related to the number of the one or more estimated morphological abnormality portions.

### Appendix 5

The drug discovery support device according to appendix 1, wherein the processor is configured to:
obtain the specimen image, the specimen image including multiple specimen images;
detect the one or more estimated morphological abnormality portions from each of the multiple specimen images; and
derive, as the determination reference information, a distribution of numerical values each representing a spatial disposition state of the one or more estimated morphological abnormality portions in a corresponding one of the multiple specimen images.

### Appendix 6

The drug discovery support device according to appendix 5, wherein
the multiple specimen images are images of a tissue specimen of multiple subjects belonging to the same group, and
the processor is configured to:
   detect an outlier from among the numerical values constituting the distribution, and thus determine whether the one or more estimated morphological abnormality portions have been overdetected and whether the one or more estimated morphological abnormality portions have been underdetected.

### Appendix 7

The drug discovery support device according to appendix 5 or appendix 6, wherein
the multiple specimen images are images of a tissue specimen of multiple subjects belonging to different groups, and
the processor is configured to:
   derive the distribution for each of the different groups; and
   detect an outlier from among the numerical values constituting one distribution of the multiple distributions derived individually for the different groups, refer to a distribution other than the one distribution, and thus determine whether the one or more estimated morphological abnormality portions have been overdetected and whether the one or more estimated morphological abnormality portions have been underdetected.

### Appendix 8

The drug discovery support device according to appendix 7, wherein the different groups are
an administration group to which the candidate substance is administered and a control group to which the candidate substance is not administered, or
an administration group to which the candidate substance is administered and an administration group to which a candidate substance identical or similar to the candidate substance is administered in a past evaluation test.

### Appendix 9

The drug discovery support device according to any one of appendix 5 to appendix 8, wherein the numerical values are each a numerical value related to the number of the one or more estimated morphological abnormality portions.

### Appendix 10

The drug discovery support device according to any one of appendix 1 to appendix 9, wherein the processor is configured to:
obtain the specimen image, the specimen image including multiple specimen images of a tissue specimen of multiple subjects belonging to different groups;
detect the one or more estimated morphological abnormality portions from each of the multiple specimen images; and
derive, as the determination reference information, for each of the different groups, a representative value of numerical values each representing a spatial disposition state of the one or more estimated morphological abnormality portions in a corresponding one of the multiple specimen images.

### Appendix 11

The drug discovery support device according to appendix 10, wherein the processor is configured to:
compare the representative value of each of the different groups with an ideal value of the numerical values that is set in advance for a corresponding one of the different groups, and thus determine whether the one or more estimated morphological abnormality portions have been overdetected and whether the one or more estimated morphological abnormality portions have been underdetected.

### Appendix 12

The drug discovery support device according to appendix 10 or appendix 11, wherein the different groups are an administration group to which the candidate substance is administered and a control group to which the candidate substance is not administered.

### Appendix 13

The drug discovery support device according to appendix 12, wherein the administration group includes multiple sub-administration groups that are different from each other in a dose of the candidate substance.

### Appendix 14

The drug discovery support device according to any one of appendix 10 to appendix 13, wherein the numerical values are each a numerical value related to the number of the one or more estimated morphological abnormality portions.

### Appendix 15

The drug discovery support device according to any one of appendix 1 to appendix 14, wherein
multiple types of artifact have a possibility of being erroneously detected as the one or more estimated morphological abnormality portions, and
the processor is configured to:
   identify a type of the artifact that has a possibility of having been erroneously detected as the one or more estimated morphological abnormality portions; and
   present, as the first cause identification reference information and the second cause identification reference information, information regarding the type to the user.

### Appendix 16

The drug discovery support device according to any one of appendix 1 to appendix 15, wherein the processor is configured to:
present, as the first cause identification reference information and the second cause identification reference information, information regarding a color of the specimen image and information regarding a color of a reference specimen image of a tissue specimen regarded to be normal to the user.

### Appendix 17

The drug discovery support device according to any one of appendix 1 to appendix 16, wherein
the specimen image includes a single specimen image,
the one or more estimated morphological abnormality portions include multiple estimated morphological abnormality portions, and
the processor is configured to:
   in response to a determination being made that the one or more estimated morphological abnormality portions have been overdetected, perform a clustering process of defining a cluster to which each of the multiple estimated morphological abnormality portions detected from the single specimen image belongs; and
   present, as the first cause identification reference information, multiple cluster images that are generated by processing the specimen image, that reflect a result of the clustering process, and that enable multiple clusters to be identified by a display format set in advance for each of the multiple clusters to the user.

### Appendix 18

The drug discovery support device according to any one of appendix 1 to appendix 17, wherein the processor is configured to:
propose to the user and/or perform a suppression process of suppressing overdetection or underdetection of the one or more estimated morphological abnormality portions.

### Appendix 19

The drug discovery support device according to appendix 18, wherein the suppression process includes at least one of:
a process of changing a detection threshold value that is to be used to detect the one or more estimated morphological abnormality portions;
a process of correcting a color of the specimen image; or
a process of changing a resolution of the specimen image in detection of the one or more estimated morphological abnormality portions.

### Appendix 20

The drug discovery support device according to any one of appendix 1 to appendix 19, wherein the processor is configured to:
propose to the user and/or perform an exclusion process of excluding, from a target of the evaluation test, a portion of an artifact that has a possibility of having been erroneously detected as the one or more estimated morphological abnormality portions.

### Appendix 21

The drug discovery support device according to any one of appendix 1 to appendix 20, wherein the processor is configured to:
handle each of multiple patch images obtained by dividing the specimen image as one of the portions; and
compare feature quantities obtained by inputting the patch images to a machine learning model with reference feature quantities obtained by inputting reference patch images of a tissue specimen regarded to be normal to the machine learning model, and thus detect the one or more estimated morphological abnormality portions.

In the technology of the present disclosure, the above-described various embodiments and/or various modifications can be combined as appropriate. The technology of the present disclosure is not limited to the above-described embodiments, and various configurations can be employed without departing from the gist. The technology of the present disclosure includes, in addition to a program, a storage medium storing the program in a non-transitory manner.

The description given above and the illustration in the drawings are detailed description of the part related to the technology of the present disclosure and are merely an example of the technology of the present disclosure. For example, the description about the configurations, functions, operations, and effects given above is the description about an example of the configurations, functions, operations, and effects of the part related to the technology of the present disclosure. Thus, it goes without saying that an unnecessary part may be deleted from, a new element may be added to, or replacement may be performed on the description given above and the illustration in the drawings without departing from the gist of the technology of the present disclosure. To avoid complexity and facilitate understanding of the part related to the technology of the present disclosure, description of common technical knowledge or the like that is not particularly necessary to implement the technology of the present disclosure is omitted in the description given above and the illustration in the drawings.

In this specification, "A and/or B" is synonymous with "at least one of A or B". That is, "A and/or B" means only A, only B, or a combination of A and B. In this specification, a concept similar to "A and/or B" is applied to three or more things connected by "and/or".

All documents, patent applications, and technical standards described in this specification are incorporated in this specification by reference to such a degree that each document, patent application, and technical standard are specifically and individually described as being incorporated by reference.

## Claims

1. A drug discovery support device comprising a processor,
the processor being configured to:
obtain a specimen image of a tissue specimen of an organ of a subject subjected to an evaluation test of a candidate substance;
detect, from among portions of the specimen image, one or more estimated morphological abnormality portions where a morphological abnormality is estimated to have occurred;
derive determination reference information from a detection result of each of the one or more estimated morphological abnormality portions;
determine, based on the determination reference information, whether the one or more estimated morphological abnormality portions have been overdetected and whether the one or more estimated morphological abnormality portions have been underdetected;
in response to a determination being made that the one or more estimated morphological abnormality portions have been overdetected, present to a user first cause identification reference information contributing to identifying a cause of the one or more estimated morphological abnormality portions having been overdetected; and
in response to a determination being made that the one or more estimated morphological abnormality portions have been underdetected, present to the user second cause identification reference information contributing to identifying a cause of the one or more estimated morphological abnormality portions having been underdetected.

2. The drug discovery support device according to claim 1, wherein the processor is configured to:
obtain the specimen image, the specimen image comprising a single specimen image;
detect the one or more estimated morphological abnormality portions from the single specimen image; and
derive, as the determination reference information, a numerical value representing a spatial disposition state of the one or more estimated morphological abnormality portions in the single specimen image.

3. The drug discovery support device according to claim 2, wherein the processor is configured to:
compare the numerical value with a determination threshold value set in advance, and thus determine whether the one or more estimated morphological abnormality portions have been overdetected and whether the one or more estimated morphological abnormality portions have been underdetected.

4. The drug discovery support device according to claim 2, wherein the numerical value is a numerical value related to the number of the one or more estimated morphological abnormality portions.

5. The drug discovery support device according to claim 1, wherein the processor is configured to:
obtain the specimen image, the specimen image comprising multiple specimen images;
detect the one or more estimated morphological abnormality portions from each of the multiple specimen images; and
derive, as the determination reference information, a distribution of numerical values each representing a spatial disposition state of the one or more estimated morphological abnormality portions in a corresponding one of the multiple specimen images.

6. The drug discovery support device according to claim 5, wherein
the multiple specimen images are images of a tissue specimen of multiple subjects belonging to the same group, and
the processor is configured to:
detect an outlier from among the numerical values constituting the distribution, and thus determine whether the one or more estimated morphological abnormality portions have been overdetected and whether the one or more estimated morphological abnormality portions have been underdetected.

7. The drug discovery support device according to claim 5, wherein
the multiple specimen images are images of a tissue specimen of multiple subjects belonging to different groups, and
the processor is configured to:
derive the distribution for each of the different groups; and
detect an outlier from among the numerical values constituting one distribution of the multiple distributions derived individually for the different groups, refer to a distribution other than the one distribution, and thus determine whether the one or more estimated morphological abnormality portions have been overdetected and whether the one or more estimated morphological abnormality portions have been underdetected.

8. The drug discovery support device according to claim 7, wherein the different groups are
an administration group to which the candidate substance is administered and a control group to which the candidate substance is not administered, or
an administration group to which the candidate substance is administered and an administration group to which a candidate substance identical or similar to the candidate substance is administered in a past evaluation test.

9. The drug discovery support device according to claim 5, wherein the numerical values are each a numerical value related to the number of the one or more estimated morphological abnormality portions.

10. The drug discovery support device according to claim 1, wherein the processor is configured to:
obtain the specimen image, the specimen image comprising multiple specimen images of a tissue specimen of multiple subjects belonging to different groups;
detect the one or more estimated morphological abnormality portions from each of the multiple specimen images; and
derive, as the determination reference information, for each of the different groups, a representative value of numerical values each representing a spatial disposition state of the one or more estimated morphological abnormality portions in a corresponding one of the multiple specimen images.

11. The drug discovery support device according to claim 10, wherein the processor is configured to:
compare the representative value of each of the different groups with an ideal value of the numerical values that is set in advance for a corresponding one of the different groups, and thus determine whether the one or more estimated morphological abnormality portions have been overdetected and whether the one or more estimated morphological abnormality portions have been underdetected.

12. The drug discovery support device according to claim 10, wherein the different groups are an administration group to which the candidate substance is administered and a control group to which the candidate substance is not administered.

13. The drug discovery support device according to claim 12, wherein the administration group includes multiple sub-administration groups that are different from each other in a dose of the candidate substance.

14. The drug discovery support device according to claim 10, wherein the numerical values are each a numerical value related to the number of the one or more estimated morphological abnormality portions.

15. The drug discovery support device according to claim 1, wherein
multiple types of artifact have a possibility of being erroneously detected as the one or more estimated morphological abnormality portions, and
the processor is configured to:
identify a type of the artifact that has a possibility of having been erroneously detected as the one or more estimated morphological abnormality portions; and
present, as the first cause identification reference information and the second cause identification reference information, information regarding the type to the user.

16. The drug discovery support device according to claim 1, wherein the processor is configured to:
present, as the first cause identification reference information and the second cause identification reference information, information regarding a color of the specimen image and information regarding a color of a reference specimen image of a tissue specimen regarded to be normal to the user.

17. The drug discovery support device according to claim 1, wherein
the specimen image comprises a single specimen image,
the one or more estimated morphological abnormality portions comprise multiple estimated morphological abnormality portions, and
the processor is configured to:
in response to a determination being made that the one or more estimated morphological abnormality portions have been overdetected, perform a clustering process of defining a cluster to which each of the multiple estimated morphological abnormality portions detected from the single specimen image belongs; and
present, as the first cause identification reference information, multiple cluster images that are generated by processing the specimen image, that reflect a result of the clustering process, and that enable multiple clusters to be identified by a display format set in advance for each of the multiple clusters to the user.

18. The drug discovery support device according to claim 1, wherein the processor is configured to:
propose to the user and/or perform a suppression process of suppressing overdetection or underdetection of the one or more estimated morphological abnormality portions.

19. The drug discovery support device according to claim 18, wherein the suppression process comprises at least one of:
a process of changing a detection threshold value that is to be used to detect the one or more estimated morphological abnormality portions;
a process of correcting a color of the specimen image; or
a process of changing a resolution of the specimen image in detection of the one or more estimated morphological abnormality portions.

20. The drug discovery support device according to claim 1, wherein the processor is configured to:
propose to the user and/or perform an exclusion process of excluding, from a target of the evaluation test, a portion of an artifact that has a possibility of having been erroneously detected as the one or more estimated morphological abnormality portions.

21. The drug discovery support device according to claim 1, wherein the processor is configured to:
handle each of multiple patch images obtained by dividing the specimen image as one of the portions; and
compare feature quantities obtained by inputting the patch images to a machine learning model with reference feature quantities obtained by inputting reference patch images of a tissue specimen regarded to be normal to the machine learning model, and thus detect the one or more estimated morphological abnormality portions.

22. A method for operating a drug discovery support device, the method comprising:
obtaining a specimen image of a tissue specimen of an organ of a subject subjected to an evaluation test of a candidate substance;
detecting, from among portions of the specimen image, one or more estimated morphological abnormality portions where a morphological abnormality is estimated to have occurred;
deriving determination reference information from a detection result of each of the one or more estimated morphological abnormality portions;
determining, based on the determination reference information, whether the one or more estimated morphological abnormality portions have been overdetected and whether the one or more estimated morphological abnormality portions have been underdetected;
in response to a determination being made that the one or more estimated morphological abnormality portions have been overdetected, presenting to a user first cause identification reference information contributing to identifying a cause of the one or more estimated morphological abnormality portions having been overdetected; and
in response to a determination being made that the one or more estimated morphological abnormality portions have been underdetected, presenting to the user second cause identification reference information contributing to identifying a cause of the one or more estimated morphological abnormality portions having been underdetected.

23. A program for operating a drug discovery support device, the program causing a computer to execute a process comprising:
obtaining a specimen image of a tissue specimen of an organ of a subject subjected to an evaluation test of a candidate substance;
detecting, from among portions of the specimen image, one or more estimated morphological abnormality portions where a morphological abnormality is estimated to have occurred;
deriving determination reference information from a detection result of each of the one or more estimated morphological abnormality portions;
determining, based on the determination reference information, whether the one or more estimated morphological abnormality portions have been overdetected and whether the one or more estimated morphological abnormality portions have been underdetected;
in response to a determination being made that the one or more estimated morphological abnormality portions have been overdetected, presenting to a user first cause identification reference information contributing to identifying a cause of the one or more estimated morphological abnormality portions having been overdetected; and
in response to a determination being made that the one or more estimated morphological abnormality portions have been underdetected, presenting to the user second cause identification reference information contributing to identifying a cause of the one or more estimated morphological abnormality portions having been underdetected.
